(19) Europäisches Patentamt
European Patent Office
Office européen des brevets

(11) **EP 4 349 993 A1**

(12) **EUROPEAN PATENT APPLICATION**

(43) Date of publication:
**10.04.2024 Bulletin 2024/15**

(21) Application number: **22199963.4**

(22) Date of filing: **06.10.2022**

(51) International Patent Classification (IPC):
*C12P 7/06* (2006.01)   *C12P 7/54* (2006.01)
*C05D 9/02* (2006.01)   *C12N 1/20* (2006.01)
*C12R 1/145* (2006.01)

(52) Cooperative Patent Classification (CPC):
**C12P 7/06; C12N 1/20; C12P 7/54;** C12R 2001/01;
C12R 2001/145

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SecondCircle ApS
2860 Søborg (DK)**

(72) Inventors:
• **DOBRESCU, Andreea-Cristina
  2860 Søborg (DK)**
• **SANTOS GIORDANI BENEVENUTI, Carolina
  2860 Søborg (DK)**
• **REDL, Stephanie
  2860 Søborg (DK)**
• **JENSEN, Torbjørn Ølshøj
  2860 Søborg (DK)**

(74) Representative: **Zacco Denmark A/S
Arne Jacobsens Allé 15
2300 Copenhagen S (DK)**

(54) **OPTIMISED FERMENTATION OF ANAEROBIC BACTERIA**

(57) The present invention provides a method for increasing the growth efficiency in an anaerobic microbial fermentation of a microorganism capable of converting $CO_2$, CO or a mixture thereof into at least one organic compound, the method comprising growing the microorganisms in a cultivation medium comprising selenium in a concentration which is at least $1.2\,\mu M$, nickel is between $3.0\,\mu M$ to $8.5\,\mu M$ and/or molybdenum is at least $1.5\,\mu M$.

EP 4 349 993 A1

**Description**

**Technical field of the invention**

The present invention relates to the fields of biotechnology, microbiology, fermentations and microbial growth and conversion of carbon dioxide or carbon monoxide into organic compounds.

**Background of the invention**

Carbon emission gasses such as carbon dioxide ($CO_2$) and carbon monoxide (CO) form during many industrial processes, mainly manufacturing, raw material mining, energy and transportation. $CO_2$ and CO have traditionally been emitted to the atmosphere as rather harmless compounds entering the global carbon cycle while environmental and health safety focus was on limiting emissions of other gasses, including toxic gasses, such as NOx, $SO_2$, methane etc. Due to increasing global population, the ongoing global warming and climate changes there is an urgent need for industrial processes which are more circular in nature, i.e. leaving little footprints and not causing large emissions disturbing global balances. No doubt, this undertaking will require many different efforts in order to timely intervene in the ongoing developments which are very fast compared to the rate of fundamentally changing the industrial processes which support man kind and our present population. Examples being substituting combustion of fossil fuels for electricity made from wind, solar, water and nuclear power, and being minimizing the emission of $CO_2$ and CO from industrial processes by alternative processes or by capturing the $CO_2$ and CO.

The capture of $CO_2$ and CO from industrial processes may fundamentally be carried out in two different ways. Firstly, $CO_2$ may be collected for the industrial process and stored so that it does not enter the atmosphere. Attempts have been made to pump the $CO_2$ into underground cavities such as in areas where oil and gas exploration have taken place. There are also large scale processes where $CO_2$ is liquefied and transported to a suitable site for long term storage. However, the concept of storing $CO_2$ does point to a less than optimal solution from a sustainability point of view. Secondly, $CO_2$ may be collected from the industrial process where it is produced and then used in another process where it is converted into some carbon-containing product which has commercial value.

WO 98/37179 discloses chemically defined media for the fermentative production of valuable compounds on an industrial scale.

WO 2010/064932 discloses optimized fermentation media for the production of alcohols by microbial fermentation of substrates comprising CO.

US 2015/0024449 discloses a process and medium for fermenting syngas wherein the selenium levels in biomass are reduced.

Redl et al., Frontiers in Microbiology (2020), Vol 10, pp 1-15 discloses a medium for the cultivation of species of *Moorella.*

Hence, there is a great need for new effective processes for converting $CO_2$ or CO from emissions from industrial processes into products having commercial value, thus serving dual purposes of eliminating $CO_2$ or CO from emissions and manufacturing a product which can ensure the process to be commercially viable.

**Summary of the invention**

It is an object of the present invention to provide processes for the conversion of $CO_2$ and CO from emissions into an organic compound. It is in particular an object of the present invention to provide an improved fermentation medium useful in a microbial process that converts $CO_2$ or CO into at least one organic compound. It is a further object of the present invention to provide an improved fermentation medium which facilitates a cost-effective process, e.g. low cost of medium and low cooling requirements. It is also an object of the present invention to overcome some of the disadvantages of the processes known in the art.

The inventors have surprisingly found that a medium having a high concentrations of selenium and molybdenum facilitates a high growth rate and high biomass yields of an acetogenic microorganism. The inventors has also found that the nickel concentration and the magnesium concentration in contrast to some prior teachings have novel and optimal intervals with respect to growth rate and yield of an acetogenic microorganism

In a first aspect the present invention provides a method for increasing the growth efficiency in an anaerobic microbial fermentation of a microorganism capable of converting $CO_2$, CO or a mixture thereof into at least one organic compound, the method comprising growing the microorganisms in a cultivation medium comprising selenium in a concentration which is at least 1.2 μM.

In a second aspect the present invention provides a method for increasing the growth efficiency in an anaerobic microbial fermentation of a microorganism converting $CO_2$, CO or a mixture thereof into at least one organic compound, the method comprising growing the microorganisms in a cultivation medium comprising nickel in a concentration which is in the range from 3.0 μM to 8.5 μM.

In a third aspect the present invention provides a method for increasing the growth efficiency in an anaerobic microbial fermentation of a microorganism converting $CO_2$, CO or a mixture thereof into at least one organic compound, the method comprising growing the microorganisms in a cultivation medium comprising molybdenum in a concentration which is at least 1.5 μM.

In a fourth aspect the present invention provides a method for switching from a phase of mainly biomass growth to a phase of mainly product formation for an anaerobic microbial fermentation of a microorganism capable of converting $CO_2$, CO or a mixture thereof into at least one organic compound, wherein said product is said at least one organic compound and said method comprising growing the microorganisms in a cultivation medium wherein the concentrations of molybdenum and nickel remains substantially constant during the switch, and the concentrations of selenium and magnesium both increases 5-15 fold during the switch.

In a fifth aspect the present invention provides the use of the method as defined in any of the first to fourth aspects for the industrial manufacture of at least one organic compound from $CO_2$, CO or a mixture thereof.

In an embodiment of the invention the microorganism is an acetogen, i.e. a microorganism having a metabolism converting $CO_2$ (with $H_2$) or CO into acetyl-coenzyme-A.

In another embodiment the microorganism is selected from the group consisting of *Clostridium*, *Moorella*, *Thermoonoerobocter, Thermoanaerobacterium, Acetogenium, Acetobocterium, Acetoonoerobium, Butyribocterium, Eubacterium, Pyrococcus, Desulfobacterium* and *Carboxydothermus.*

The invention is further summarized in the following list of items :

1. A method for increasing the growth efficiency in an anaerobic microbial fermentation of a microorganism capable of converting $CO_2$, CO or a mixture thereof into at least one organic compound, the method comprising growing the microorganisms in a cultivation medium comprising selenium in a concentration which is at least 1.2 μM.

2. A method for increasing the growth efficiency in an anaerobic microbial fermentation of a microorganism converting $CO_2$, CO or a mixture thereof into at least one organic compound, the method comprising growing the microorganisms in a cultivation medium comprising nickel in a concentration which is in the range from 3.0 μM to 8.5 μM.

3. A method for increasing the growth efficiency in an anaerobic microbial fermentation of a microorganism converting $CO_2$, CO or a mixture thereof into at least one organic compound, the method comprising growing the microorganisms in a cultivation medium comprising molybdenum in a concentration which is at least 1.5 μM.

4. The method according to any of the preceding items, wherein said cultivation medium comprises selenium in a concentration which is at least 1.3 μM, at least 2 μM, at least 3 μM, or at least 4 μM.

5. The method according to any of the preceding items, wherein said cultivation medium comprises selenium in a concentration which is in the range from 2.0 μM to 8 μM, in the range from 3.0 μM to 7 μM, in the range from 4 μM to 6 μM or in the range from 4.4 μM to 5.6 μM.

6. The method according to any of the preceding items, wherein said cultivation medium comprises selenium in a concentration which is in the range from 4.4 μM to 5.6 μM.

7. The method according to any of the preceding items, wherein said microorganism grows on a hexose as the main carbon source, such as fructose or glucose.

8. The method according to any of the preceding items, wherein said cultivation medium comprises selenium in a concentration which is at least 5.0 μM, at least 10 μM, at least 20 μM, or at least 30 μM.

9. The method according to any of the preceding items, wherein said cultivation medium comprises selenium in a concentration which is in the range from 5.0 μM to 70 μM, in the range from 10 μM to 60 μM, in the range from 20 μM to 50 μM or in the range from 30 μM to 50 μM.

10. The method according to any of items 1-6 and 8-9, wherein said microorganism grows on a gas as the main carbon source, such as $CO_2$ or CO.

11. The method according to any of the preceding items, wherein said cultivation medium comprises nickel in a concentration which is in the range from 4.0 μM to 8.0 μM, in the range from 5.0 μM to 7.0 μM or in the range from 5.5 μM to 7.0 μM.

12. The method according to any of the preceding items, wherein said cultivation medium comprises molybdenum in a concentration which is at least 1.7 $\mu$M, at least 2.0 $\mu$M, or at least 2.2 $\mu$M.

13. The method according to any of the preceding items, wherein said cultivation medium comprises molybdenum in a concentration which is in the range from 1.5 $\mu$M to 20 $\mu$M, in the range from 1.5 $\mu$M to 10 $\mu$M or in the range from 2.0 $\mu$M to 5 $\mu$M.

14. The method according to any of the preceding items, wherein the concentration of magnesium in said cultivation medium is in the range from 0.05 mM to 40 mM.

15. The method according to item 14, wherein said microorganism grows on a hexose as the main carbon source and the concentration of magnesium in said cultivation medium is in the range from 0.05 mM to 1.3 mM, from 0.10 mM to 0.8 mM, or from 0.15 mM to 0.30 mM.

16. The method according to item 14, wherein said microorganism grows on a gas such as $CO_2$ or C0 as the main carbon source and the concentration of magnesium in said cultivation medium is in the range from 10 mM to 40 mM, from 15 mM to 35 mM, or from 18 mM to 27 mM.

17. The method according to any of the preceding items, wherein said microorganism is capable of converting $CO_2$ into at least one organic compound.

18. The method according to any of the preceding items, wherein said microorganism is capable of converting CO into at least one organic compound.

19. The method according to any of the preceding items, wherein said at least one organic compound is a $C_{1-6}$-compound.

20. The method according to item 9a, wherein said $C_{1-6}$-compound is a $C_{1-6}$-alcohol, a $C_{1-6}$-carboxylic acid or a $C_{1-6}$-ketone.

21. The method according to any of the preceding items, wherein said microorganism is an acetogen.

22. The method according to any of the preceding items, wherein said microorganism is selected from the group consisting of *Clostridium*, *Moorella*, *Thermoonoerobocter*, *Thermoanaerobacterium*, *Acetogenium*, *Acetobocterium*, *Acetoonoerobium*, *Butyribocterium*, *Eubacterium*, *Pyrococcus*, *Desulfobacterium* and *Carboxydothermus.*

23. The method according to item 22, wherein said microorganism is *Moorella* or *Thermoonoerobocter.*

24. The method according to item 23, wherein said microorganism is *Moorella thermoacetica* or *Moorella thermoautotrophica.*

25. The method according to any of the preceding items, wherein said at least one organic compound comprises acetate.

26. The method according to any of the preceding items, wherein said at least one organic compound comprises ethanol.

27. The method according to any of the preceding items, wherein said anaerobic microbial fermentation is a biomass propagation step.

28. The method according to any of items 1-9 and 11-27, wherein said cultivation medium comprises a hexose as the main carbon source.

29. The method according to item 28, wherein said hexose is fructose or glucose.

30. The method according to any of items 1-26, wherein said anaerobic microbial fermentation is a production of said at least one organic compound from said cultivation medium and a gas comprising $CO_2$, CO or a mixture thereof.

31. The method according to item 30, wherein said gas is syngas, i.e. a mixture comprising CO and $H_2$.

32. The method according to any of the preceding items, wherein said cultivation medium comprises a nutrient selected from the group consisting of Al, Mn, Fe, Co, Zn, and Cu.

33. The method according to any of the preceding items, wherein said cultivation medium comprises Mn, Fe, Co, Zn and Cu.

34. The method according to any of the preceding items, wherein said cultivation medium comprises a nutrient selected from the group consisting of biotin, folic acid, pyridoxine, thiamine, riboflavin, nicotinic acid, D-pantothenate, vitamin B12, para-amino-benzoic acid and thiotic acid.

35. The method according to any of the preceding items, wherein said cultivation medium comprises biotin, folic acid, pyridoxine, thiamine, riboflavin, nicotinic acid, D-pantothenate, vitamin B12, para-amino-benzoic acid and thiotic acid.

36. The method according to any of the preceding items, wherein the growth efficiency is the specific growth rate.

37. The method according to any of the preceding items, wherein the growth efficiency is the biomass yield per carbon substrate provided in said cultivation medium.

38. The method according to any of the preceding items, wherein said microbial fermentation is a batch fermentation or a fed-batch fermentation.

39. The method according to any of the preceding items, wherein said microbial fermentation is a continuous fermentation.

40. The method according to any of the preceding items, wherein said microbial fermentation is an industrial scale process.

41. The method according to item 40, wherein said industrial scale process is a fermentation in at least 5 $m^3$ scale, at least 30 $m^3$ scale or at least 80 $m^3$ scale.

42. A method for switching from a phase of mainly biomass growth to a phase of mainly product formation for an anaerobic microbial fermentation of a microorganism capable of converting $CO_2$, CO or a mixture thereof into at least one organic compound, wherein said product is said at least one organic compound and said method comprising growing the microorganisms in a cultivation medium wherein the concentrations of molybdenum and nickel remains substantially constant during the switch, and the concentrations of selenium and magnesium both increases 5-15 fold during the switch.

43. The method according to item 42, wherein the concentrations of selenium and magnesium both increases 7-13 fold during the switch.

44. The method according to any of items 42-43, wherein the concentration of selenium in the cultivation medium used before the switch is in the range from 2.0 $\mu M$ to 8 $\mu M$, and the concentration of magnesium in the cultivation medium used before the switch is in the range from 0.15 mM to 0.30 mM.

45. The method according to any of items 42-44, wherein the concentration of molybdenum in the cultivation medium used before the switch is at least 1.5 $\mu M$, and the concentration of nickel in the cultivation medium used before the switch is in the range from 3.0 $\mu M$ to 8.5 $\mu M$.

46. The method according to any of items 42-45, wherein said phase of mainly biomass growth takes place with a cultivation medium wherein one or more hexoses is the main carbon source.

47. The method according to any of items 42-46, wherein said phase of mainly product formation takes place with a cultivation medium wherein a gas such as $CO_2$ or CO is the main carbon source.

48. The method according to any of items 42-47, wherein said phase of mainly biomass growth is conducted as a batch or fed-batch fermentation.

49. The method according to any of items 42-48, wherein said phase of mainly product formation is conducted as a fed-batch fermentation or a continuous fermentation.

50. The method according to any of items 42-49, wherein said switch of cultivation medium lasts less than 5 hours, less than 2 hours, less than 1 hour or less than 30 minutes.

51. Use of the method as defined in any of items 1-50 for the industrial manufacture of at least one organic compound from $CO_2$, CO or a mixture thereof.

52. The use according to item 51, wherein said at least one organic compound comprises a $C_{1-6}$-compound, such as a $C_{1-6}$-alcohol, a $C_{1-6}$-carboxylic acid or a $C_{1-6}$-ketone.

53. The use according to item 52, wherein said at least one organic compound comprises acetate.

54. The use according to any of items 51-53, wherein said at least one organic compound comprises ethanol.

## Brief description of the figures

Figure 1. Significance of the compounds tested using Plackett-Burman design on the growth of *Moorella thermoacetica* on fructose. A t-value above 1.65 or below -1.65 indicates a significant effect at a 90% confidence level.

Figure 2. Generated response surface plot showing the influence of yeast extract and $MgCl_2$ concentrations on biomass production after 24 hours of fermentation on fructose.

Figure 3. Significance of the compounds tested using Plackett-Burman design on the growth of *Moorella thermoacetica* on fructose. A t-value above 1.65 or below -1.65 indicates a significant effect at a 90% confidence level.

Figure 4. Generated response surface plots showing the influence of $Na_2MoO_4$, $NiCl_2$ and $Na_2SeO_4$ concentrations on biomass production after 24 hours of fermentation on fructose.

Figure 5. Growth patterns of *Moorella thermoacetica* in the improved and original media using fructose as carbon source. The cell density is measured using a real-time monitoring device.

Figure 6. Acetate concentrations achieved after 63 hours of fermentation with *Moorella thermoacetica* in the improved and original media using fructose as carbon source.

Figure 7. Significance of the compounds tested using Plackett-Burman design on the growth of *Moorella thermoacetica* on $CO_2$. A t-value above 1.65 or below -1.65 indicates a significant effect at a 90% confidence level.

Figure 8. Generated response surface plot showing the influence of $MgCl_2$ concentrations on biomass production after 24 hours of fermentation using $CO_2$ as carbon source.

Figure 9. Significance of the compounds tested using Plackett-Burman design on the growth of *Moorella thermoacetica* on $CO_2$. A t-value above 1.65 or below -1.65 indicates a significant effect at a 90% confidence level.

Figure 10. Generated response surface plot showing the influence of $Na_2SeO_4$ and $Na_2MoO_4$ concentrations on biomass production after 24 hours of fermentation using $CO_2$ as carbon source.

Figure 11. Absorbance indicating biomass concentration in the improved and original media after 24 hours of fermentation using $CO_2$ as carbon source.

## Detailed description of the invention

Unless specifically defined herein, all technical and scientific terms used have the same meaning as commonly understood by a skilled artisan in the fields of biotechnology, microbiology, fermentations and microbial growth and product formation.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of biotechnology, microbiology, fermentation technology which are within the skill of the art. Such techniques are explained fully in the literature.

Cultivation medium

In a first aspect the present invention provides a method for increasing the growth efficiency in an anaerobic microbial fermentation of a microorganism capable of converting $CO_2$, CO or a mixture thereof into at least one organic compound, the method comprising growing the microorganisms in a cultivation medium comprising selenium in a concentration which is at least 1.2 $\mu$M.

In a second aspect the present invention provides a method for increasing the growth efficiency in an anaerobic microbial fermentation of a microorganism converting $CO_2$, CO or a mixture thereof into at least one organic compound, the method comprising growing the microorganisms in a cultivation medium comprising nickel in a concentration which is in the range from 3.0 $\mu$M to 8.5 $\mu$M.

In a third aspect the present invention provides a method for increasing the growth efficiency in an anaerobic microbial fermentation of a microorganism converting $CO_2$, CO or a mixture thereof into at least one organic compound, the method comprising growing the microorganisms in a cultivation medium comprising molybdenum in a concentration which is at least 1.5 $\mu$M.

It is to be understood that in a cultivation medium the selenium concentration which is at least 1.2 $\mu$M, may be combined with nickel in a concentration which is in the range from 3.0 $\mu$M to 8.5 $\mu$M and with molybdenum in a concentration which is at least 1.5 $\mu$M. In fact, it is believed that this combination of these three metals is a preferred embodiment.

In an embodiment the cultivation medium comprises selenium in a concentration which is at least 1.3 $\mu$M, at least 2 $\mu$M, at least 3 $\mu$M, or at least 4 $\mu$M. In another embodiment the cultivation medium comprises selenium in a concentration which is in the range from 2.0 $\mu$M to 8 $\mu$M, in the range from 3.0 $\mu$M to 7 $\mu$M, in the range from 4 $\mu$M to 6 $\mu$M or in the range from 4.4 $\mu$M to 5.6 $\mu$M. In another embodiment the cultivation medium comprises selenium in a concentration which is in the range from 4.4 $\mu$M to 5.6 $\mu$M. In yet another embodiment the cultivation medium comprises selenium in a concentration which is at least 5.0 $\mu$M, at least 10 $\mu$M, at least 20 $\mu$M, or at least 30 $\mu$M. In yet another embodiment the cultivation medium comprises selenium in a concentration which is in the range from 5.0 $\mu$M to 70 $\mu$M, in the range from 10 $\mu$M to 60 $\mu$M, in the range from 20 $\mu$M to 50 $\mu$M or in the range from 30 $\mu$M to 50 $\mu$M.

In another embodiment the cultivation medium comprises nickel in a concentration which is in the range from 4.0 $\mu$M to 8.0 $\mu$M, in the range from 5.0 $\mu$M to 7.0 $\mu$M or in the range from 5.5 $\mu$M to 7.0 $\mu$M.

In another embodiment the cultivation medium comprises molybdenum in a concentration which is at least 1.7 $\mu$M, at least 2.0 $\mu$M, or at least 2.2 $\mu$M. In another embodiment the cultivation medium comprises molybdenum in a concentration which is in the range from 1.5 $\mu$M to 20 $\mu$M, in the range from 1.5 $\mu$M to 10 $\mu$M or in the range from 2.0 $\mu$M to 5 $\mu$M.

In another embodiment the concentration of magnesium in said cultivation medium is in the range from 0.05 mM to 40 mM. In another embodiment the microorganism grows on a hexose as the main carbon source and the concentration of magnesium in said cultivation medium is in the range from 0.05 mM to 1.3 mM, from 0.10 mM to 0.8 mM, or from 0.15 mM to 0.30 mM. In yet another embodiment the microorganism grows on a gas such as $CO_2$ or CO as the main carbon source and the concentration of magnesium in said cultivation medium is in the range from 10 mM to 40 mM, from 15 mM to 35 mM, or from 18 mM to 27 mM.

Different carbon sources may be used for propagating the microorganisms prior to the product formation phase, and such carbon sources may in some embodiments also be added during the product formation phase where $CO_2$, CO or a mixture thereof is converted into at least one organic compound.

In an embodiment the microorganism grows on a hexose as the main carbon source, such as fructose or glucose. In another embodiment the microorganism grows on $CO_2$, CO or a mixture thereof as the main carbon source.

Other useful medium components are apparent from the Examples and the items of the present application.

Microorganisms

It is to be understood that a range of different microorganisms can be used in the present invention as long as they are capable of converting $CO_2$, CO or a mixture thereof into at least one organic compound. Typically useful microorganisms are anaerobic microorganisms.

In an embodiment the microorganism is an acetogen.

Different acetogens may be used in the present invention. In some embodiments the microorganism is capable of converting $CO_2$ into at least one organic compound. In some embodiment the microorganism is capable of converting CO into at least one organic compound.

In one embodiment the at least one organic compound is a $C_{1-6}$-compound.

In another embodiment the $C_{1-6}$-compound is a $C_{1-6}$-alcohol, a $C_{1-6}$-carboxylic acid or a $C_{1-6}$-ketone.

Other useful microorganisms are selected from the group consisting of *Clostridium, Moorella, Thermoonoerobocter, Thermoanaerobacterium, Acetogenium, Acetobocterium, Acetoonoerobium, Butyribocterium, Eubacterium, Pyrococcus, Desulfobacterium* and *Carboxydothermus.*

In an embodiment the microorganism is *Moorella* or *Thermoonoerobocter.*

In another embodiment the microorganism is *Moorella thermoacetica* or *Moorella thermoautotrophica.*

The microorganism used in the invention may be a naturally occurring microorganisms, it may be a microorganisms optimized through selection, and it may be a microorganism which is genetically engineered, e.g. for increased product formation or for rendering it capable of synthesizing a specific organic compound of interest.

In an embodiment the at least one organic compound comprises acetate. In another embodiment the at least one organic compound is mainly acetate.

In another embodiment the at least one organic compound comprises ethanol. In another embodiment the at least one organic compound is mainly ethanol.

Growth efficiency of a microorganism indicates how well the growth is under certain fermentation conditions. In one embodiment the growth efficiency is the specific growth rate. In another embodiment the growth efficiency is the biomass yield per carbon substrate provided in said cultivation medium.

## Examples

### Example 1

This experiment describes the growth improvement of *Moorella thermoacetica* on fructose, achieved by modifying the composition of the growth medium using a statistical approach. Initially, Plackett-Burman design is used to identify which components have a significant effect on cell growth. Then, response surface methodology (RSM) is used to determine improved concentrations of those components. The factors tested in this experiment are the following eight components: yeast extract, $NH_4Cl$, trace elements, vitamins, $MgCl_2$, $CaCl_2$, $KH_2PO_4$, and NaCl.

The following stock solutions are prepared and sterilised: fructose (180 g/L), yeast extract (100 g/L), $NH_4Cl$ (40 g/L), $MgCl_2 \cdot 6H_2O$ (20 g/L), $CaCl_2 \cdot 2H_2O$ (10 g/L), $KH_2PO_4$ (20 g/L), NaCl (20 g/L), MES monohydrate (200 g/L, pH 6.5), cysteine-HCl (100 mM) and resazurin (0.01 g/L). The vitamin solution is filter-sterilised and contains biotin (2 mg/L), folic acid (2 mg/L), pyridoxine hydrochloride (10 mg/L), thiamine-HCl (5 mg/L), riboflavin (5 mg/L), nicotinic acid (5 mg/L), calcium-D-(+)-pantothenate (5 mg/L), Vitamin B12 (0.5 mg/L), p-aminobenzoic acid (5 mg/L), thioctic acid (5 mg/L). The trace element solution is prepared by dissolving nitrilotriacetic acid (2 g/L) in water while increasing the pH to 6.0 with 2 M KOH, after which $MnSO_4 \cdot H_2O$ (1 g/L), $Fe(SO_4)_2(NH_4)_2 \cdot 6H_2O$ (0.8 g/L), $CoCl_2 \cdot 6H_2O$ (0.2 g/L), $ZnSO_4 \cdot 7H_2O$ (0.2 g/L), $CuCl_2 \cdot 2H_2O$ (20 mg/L), $NiCl_2 \cdot 6H_2O$ (20 mg/L), $Na_2MoO_4 \cdot 2H_2O$ (20 mg/L), $Na_2SeO_4$ (20 mg/L), $Na_2WO_4 \cdot 2H_2O$ (20 mg/L) are added.

Each of the eight components are screened on two levels (Table 1). The Placket-Burman matrix, describing the final concentration of each factor in the 12 experiments performed, is generated with the statistical program JMP (Table 2). The experimental procedure is performed in an anaerobic chamber to guarantee an anoxic environment. Anoxic and sterile stock solutions are mixed to reach final concentrations as indicated in Table 2, along with MES monohydrate (final concentration 20 g/L; pH 6.5), resazurin (0.001 g/L), fructose (10.8 g/L) and cysteine-HCl (1 mM). 4.9 mL of each medium mix is aliquoted to 3 wells of a 24-well deep-well plate, and 0.1 mL inoculum ($OD_{600}$ = 3.62) is added to all wells. The plates are placed in an anoxic box, flushed with $N_2/CO_2$ (80/20) and, with a final pressure of 0.8 bars, incubated at 60 °C without shaking. After 24 hours, the plates are removed from the incubator and the culture of each well is transferred to a 96-well microtiter plate. The absorbance is read in a spectrophotometer at 600 nm (Table 3).

**Table 1. The two levels selected for each factor tested using Plackett-Burman design and fructose as carbon source.**

| Level | Yeast extract (g/L) | NH$_4$Cl (g/L) | TE (% v/v) | Vitamins (% v/v) | MgCl$_2$· 6H$_2$O (g/L) | CaCl$_2$· 2H$_2$O (g/L) | KH$_2$PO$_4$ (g/L) | NaCl (g/L) |
|---|---|---|---|---|---|---|---|---|
| High (+) | 2 | 1 | 2 | 2 | 0.5 | 0.1 | 0.5 | 1 |
| Low (-) | 0.5 | 0.4 | 1 | 1 | 0.1 | 0.05 | 0.1 | 0.1 |

**Table 2. Plackett-Burman matrix used to examine which of the factors tested have a significant effect on the growth of *Moorella thermoacetica* on fructose**

| Run | Pattern | Yeast extract (g/L) | NH$_4$Cl (g/L) | TE (% v/v) | Vitamins (% v/v) | MgCl$_2$· 6H$_2$O (g/L) | CaCl$_2$· 2H$_2$O (g/L) | KH$_2$PO$_4$ (g/L) | NaCl (g/L) |
|---|---|---|---|---|---|---|---|---|---|
| 1 | +++++++ | 2 | 1 | 2 | 2 | 0.5 | 0.1 | 0.5 | 1 |
| 2 | +---+--+ | 2 | 0.4 | 1 | 1 | 0.5 | 0.05 | 0.1 | 1 |
| 3 | +-+++--- | 2 | 0.4 | 2 | 2 | 0.5 | 0.05 | 0.1 | 0.1 |
| 4 | --+--+-+ | 0.5 | 0.4 | 2 | 1 | 0.1 | 0.1 | 0.1 | 1 |
| 5 | ++---+-- | 2 | 1 | 1 | 1 | 0.1 | 0.1 | 0.1 | 0.1 |
| 6 | --+-+++- | 0.5 | 0.4 | 2 | 1 | 0.5 | 0.1 | 0.5 | 0.1 |
| 7 | -+++---+ | 0.5 | 1 | 2 | 2 | 0.1 | 0.05 | 0.1 | 1 |
| 8 | ---+--+- | 0.5 | 0.4 | 1 | 2 | 0.1 | 0.05 | 0.5 | 0.1 |
| 9 | +--+-+++ | 2 | 0.4 | 1 | 2 | 0.1 | 0.1 | 0.5 | 1 |
| 10 | -+-+++-- | 0.5 | 1 | 1 | 2 | 0.5 | 0.1 | 0.1 | 0.1 |
| 11 | +++---+- | 2 | 1 | 2 | 1 | 0.1 | 0.05 | 0.5 | 0.1 |
| 12 | -+--+-++ | 0.5 | 1 | 1 | 1 | 0.5 | 0.05 | 0.5 | 1 |

**Table 3. Absorbance read-outs indicating biomass concentration after 24 hours of fermentation in the Plackett-Burman experiment**

| Run | Pattern | Absorbance at 600 nm (a.u.) | | |
|---|---|---|---|---|
| | | Replicate 1 | Replicate 2 | Replicate 3 |
| 1 | +++++++ | 0.825 | 0.720 | 0.784 |
| 2 | +---+--+ | 0.839 | 0.842 | 0.806 |
| 3 | +-+++--- | 0.891 | 0.769 | 0.702 |
| 4 | --+--+-+ | 0.750 | 0.776 | 0.772 |
| 5 | ++---+-- | 0.862 | 0.818 | 0.814 |
| 6 | --+-+++- | 0.691 | 0.702 | 0.674 |
| 7 | -+++---+ | 0.818 | 0.742 | 0.688 |
| 8 | ---+--+- | 0.829 | 0.922 | 0.910 |
| 9 | +--+-+++ | 0.864 | 0.809 | 0.878 |
| 10 | -+-+++-- | 0.711 | 0.692 | 0.705 |
| 11 | +++---+- | 0.824 | 0.839 | 0.887 |

(continued)

| Run | Pattern | Absorbance at 600 nm (a.u.) | | |
|---|---|---|---|---|
| | | Replicate 1 | Replicate 2 | Replicate 3 |
| 12 | -+--+-++ | 0.702 | 0.709 | 0.700 |

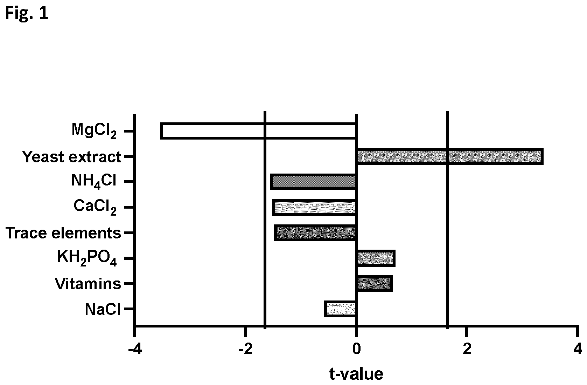

The results of the Plackett-Burman experiment are analysed with JMP (Figure 1). Yeast extract and $MgCl_2$ are the only components with a significant effect on cell growth. $MgCl_2$ has a significantly negative effect on cell growth, while yeast extract has a significantly positive effect. Based on these results, a response surface methodology (RSM) experiment is performed to optimise the concentration of the two components.

The response surface experiment is performed as a central composite design with 2 centre points and an axial value of 1.414, and the respective matrix is generated by JMP (Table 4). The high and low values are chosen based on the Plackett-Burman experiment, and the centre and axial points are calculated by JMP. The axial and centre points are used to determine the curvature of the response surface. In the response matrix, -, +, 0, a and A represent the low value, high value, centre point, lower axial point and upper axial point, respectively. Yeast extract is shown to have a positive effect, so the low value for RSM is chosen as the high value in Plackett-Burman (2 g/L). The high value is arbitrarily chosen as 10 g/L. Conversely, $MgCl_2 \cdot 6H_2O$ has a negative effect, so the low value in Placket-Burman is chosen as the high value in the response surface experiment (0.1 g/L). The low value is chosen as 0.02 g/L.

**Table 4. Response surface matrix used to optimise the concentrations of yeast extract and $MgCl_2$ for growth of *Moorella* thermoacetica on fructose**

| Run | Pattern | Yeast extract (g/L) | $MgCl_2 \cdot 6H_2O$ (g/L) |
|---|---|---|---|
| 1 | a0 | 0.34 | 0.06 |
| 2 | -- | 2.00 | 0.02 |
| 3 | -+ | 2.00 | 0.10 |
| 4 | 0a | 6.00 | 0.003 |
| 5 | 00 | 6.00 | 0.06 |
| 6 | 00 | 6.00 | 0.06 |
| 7 | 0A | 6.00 | 0.12 |
| 8 | +- | 10.00 | 0.02 |
| 9 | ++ | 10.00 | 0.10 |
| 10 | A0 | 11.66 | 0.06 |

The entire procedure of the response surface experiment is performed in an anaerobic chamber. Ten medium solutions are prepared according to the response surface matrix (Table 4), where the concentration of yeast extract and $MgCl_2 \cdot 6H_2O$ is varied by adding the stock solutions to a medium containing MES monohydrate (final concentration 20 g/L; pH 6.5), $NH_4Cl$ (0.4 g/L), $KH_2PO_4$ (0.5 g/L), trace elements (1 % v/v), vitamins (1 % v/v), $CaCl_2 \cdot 2H_2O$ (0.05 g/L), NaCl (0.4 g/L), fructose (5.4 g/L), resazurin (0.001 g/L) and cysteine-HCl (1 mM). 4.9 mL of each solution is aliquoted to 3 wells of a 24-well deep-well plate, and 0.1 mL inoculum ($OD_{600}$ = 0.343) is added to all wells. The plates are placed in an anoxic box flushed with $N_2/CO_2$ (80/20), with a final pressure of 0.8 bars. The box is incubated at 60 °C without shaking. After 24 hours, the plates are removed from the incubator and each solution is transferred to a 96-well microtiter plate. The absorbance is read in a spectrophotometer at 630 nm (Table 5).

**Table 5 Absorbance read-outs indicating biomass concentration of the RSM experiment on yeast extract and $MgCl_2$**

| Run | Pattern | Absorbance at 630 nm (a.u.) | | |
|---|---|---|---|---|
| | | Replicate 1 | Replicate 2 | Replicate 3 |
| 1 | a0 | 0.518 | 0.607 | 0.466 |
| 2 | -- | 0.463 | 0.536 | 0.444 |

(continued)

| Run | Pattern | Absorbance at 630 nm (a.u.) | | |
|-----|---------|----------------------------|---|---|
|     |         | Replicate 1 | Replicate 2 | Replicate 3 |
| 3 | -+ | 0.485 | 0.464 | 0.499 |
| 4 | 0a | 0.659 | 0.696 | 0.698 |
| 5 | 00 | 0.77 | 0.755 | 0.741 |
| 6 | 00 | 0.659 | 0.747 | 0.776 |
| 7 | 0A | 0.761 | 0.741 | 0.767 |
| 8 | +- | 0.883 | 0.896 | 0.909 |
| 9 | ++ | 0.727 | 0.708 | 0.703 |
| 10 | A0 | 0.751 | 0.735 | 0.759 |

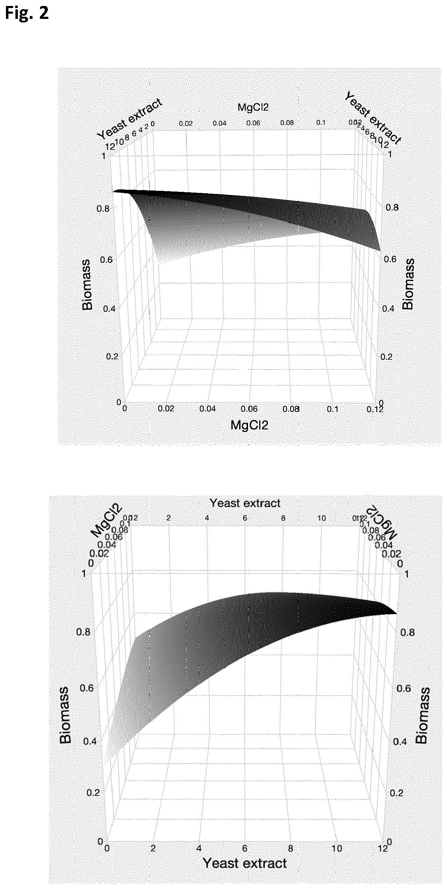

The results are analysed by JMP, which generates a second-degree polynomial equation (Equation 1) and the corresponding response surface plot (Figure 2). The analysis shows that, when *Moorella thermoacetica* is grown on fructose, the biomass concentration increases with increased yeast extract and decreased $MgCl_2$ concentrations.

$$0.738734065270186 +$$

$$+ 0.119117490895689 * ((Yeast\ extract - 6) / 4) +$$

$$+ -0.00856188505625192 * ((MgCl_2 - 0.06) / 0.04) +$$

$$+ ((Yeast\ extract - 6) / 4) * (((MgCl_2 - 0.06) / 0.04) * -0.04625) +$$

$$+ ((Yeast\ extract - 6) / 4) * (((Yeast\ extract - 6) / 4) * -0.0595951861901537) +$$

$$+ ((MgCl_2 - 0.06) / 0.04) * (((MgCl_2 - 0.06) / 0.04) * -0.0161111425222695)$$

**Equation 1. Second degree polynomial equation resulted from the RSM experiment on yeast extract and $MgCl_2$**

**Example 2**

This experiment describes the statistical optimisation of selected trace elements for improved growth of *Moorella thermoacetica* on fructose. Plackett-Burman design is used to identify which components have a significant effect on cell growth and response surface methodology (RSM) is used to determine improved concentrations of those components. The factors tested in this experiment are the following 12 components: $KAl(SO_4)_2$, $CoCl_2$, $CuCl_2$, $H_3BO_3$, $FeSO_4$, $MnSO_4$, $NiCl_2$, nitrilotriacetic acid, $Na_2SeO_4$, $Na_2MoO_4$, $Na_2WO_4$ and $ZnSO_4$.

The following stock solutions are prepared and sterilised: fructose (500 g/L), yeast extract (100 g/L), $NH_4Cl$ (40 g/L), $MgCl_2 \cdot 6H_2O$ (20 g/L), $CaCl_2 \cdot 2H_2O$ (10 g/L), $KH_2PO_4$ (20 g/L), NaCl (20 g/L), MES monohydrate (200 g/L; pH 6.5), cysteine-HCl (100 mM), resazurin (0.01 g/L), nitrilotriacetic acid (1 g/L; pH 6 adjusted with KOH), $MnSO_4 \cdot H_2O$ (20 g/L), $Fe(SO_4) \cdot 7H_2O$ (14 g/L), $CoCl_2 \cdot 6H_2O$ (10 g/L), $ZnSO_4 \cdot 7H_2O$ (4 g/L), $KAl(SO_4)_2 \cdot 12H_2O$ (1 g/L), $CuCl_2 \cdot 2H_2O$ (1 g/L), $NiCl_2 \cdot 6H_2O$ (1 g/L), $Na_2MoO_4 \cdot 2H_2O$ (1 g/L), $Na_2SeO_4$ (1 g/L) and $Na_2WO_4 \cdot 2H_2O$ (1 g/L). The vitamin solution is filter-sterilised and contains biotin (2 mg/L), folic acid (2 mg/L), pyridoxine hydrochloride (10 mg/L), thiamine-HCl (5 mg/L), riboflavin (5 mg/L), nicotinic acid (5 mg/L), calcium-D-(+)-pantothenate (5 mg/L), Vitamin B12 (0.5 mg/L), p-aminobenzoic acid (5 mg/L), thioctic acid (5 mg/L).

The initial screening is performed on two levels (Table 6). The Placket-Burman matrix, describing the final concentration of each factor in the 20 experiments performed, is generated with the statistical program JMP (Table 7). The experimental procedure is performed in an anaerobic chamber to guarantee an anoxic environment. Anoxic and sterile stock solutions are mixed to reach final concentrations as indicated in Table 7, along with MES monohydrate (final concentration 20 g/L; pH 6.5), fructose (5 g/L), yeast extract (0.5 g/L), $NH_4Cl$ (0.4 g/L), $MgCl_2 \cdot 6H_2O$ (0.33 g/L), $CaCl_2 \cdot 2H_2O$ (0.05 g/L), $KH_2PO_4$ (0.5 g/L), NaCl (0.4 g/L), vitamin solution (1 % v/v), resazurin (0.001 g/L) and cysteine-HCl (1 mM). 490 $\mu$L of each medium mix is aliquoted to 3 wells of a 96-well deep-well plate, and 10 $\mu$L inoculum ($OD_{600}$ = 0.6) is added to all wells. The plates are placed in an anoxic box, flushed with $N_2/CO_2$ (80/20) and, with a final pressure of 0.8 bars, incubated at 60 °C without shaking. After 24 hours, the plates are removed from the incubator and the culture of each well is transferred to a 96-well microtiter plate. The absorbance is read in a spectrophotometer at 630 nm (Table 8).

**Table 6. The two levels selected for each factor tested using Plackett-Burman design and fructose as carbon source**

| Level | KAl(SO₄)₂·12H₂O (mg/L) | CoCl₂·6H₂O (mg/L) | CuCl₂·2H₂O (mg/L) | H₃BO₃ (mg/L) | FeSO₄·7H₂O (mg/L) | MnSO₄·H₂O (mg/L) | Na₂MoO₄·2H₂O (mg/L) | NiCl₂·6H₂O (mg/L) | NTA* (mg/L) | Na₂SeO₄ (mg/L) | Na₂WO₄·2H₂O (mg/L) | ZnSO₄·7H₂O (mg/L) |
|---|---|---|---|---|---|---|---|---|---|---|---|---|
| High (+) | 0.2 | 2 | 0.2 | 0.2 | 2.24 | 10 | 0.2 | 0.5 | 30 | 0.2 | 0.2 | 2 |
| Low (-) | 0.05 | 0.5 | 0.05 | 0.05 | 0.56 | 2.5 | 0.05 | 0.1 | 7.5 | 0.05 | 0.05 | 0.5 |

*NTA = nitrilotriacetic acid

**Table 7. Plackett-Burman matrix used to examine which of the factors tested have a significant effect on the growth of Moorella thermoacetica on fructose**

| Level | Pattern | KAl(SO₄)₂·12H₂O (mg/L) | CoCl₂·6H₂O (mg/L) | CuCl₂·2H₂O (mg/L) | H₃BO₃ (mg/L) | FeSO₄·7H₂O (mg/L) | MnSO₄·H₂O (mg/L) | Na₂MoO₄·2H₂O (mg/L) | NiCl₂·6H₂O (mg/L) | NTA* (mg/L) | Na₂SeO₄ (mg/L) | Na₂WO₄·2H₂O (mg/L) | ZnSO₄·7H₂O (mg/L) |
|---|---|---|---|---|---|---|---|---|---|---|---|---|---|
| 1 | ---- ++-- +--+ | 0.05 | 0.5 | 0.05 | 0.05 | 2.24 | 10 | 0.05 | 0.1 | 30 | 0.05 | 0.05 | 2 |
| 2 | ---+ +--+ --++ | 0.05 | 0.5 | 0.05 | 0.2 | 2.24 | 2.5 | 0.05 | 0.5 | 7.5 | 0.05 | 0.2 | 2 |

| | | | | | | | | | | | | | | |
|---|---|---|---|---|---|---|---|---|---|---|---|---|---|---|
| 3 | --+- -+++ +-+- | 0.05 | 0.5 | 0.2 | 0.05 | 0.56 | 10 | 0.2 | 0.5 | 30 | 0.05 | 0.2 | 0.5 |
| 4 | --++ --+- -+++ | 0.05 | 0.5 | 0.2 | 0.2 | 0.56 | 2.5 | 0.2 | 0.1 | 7.5 | 0.2 | 0.2 | 2 |
| 5 | --++ ++-+ -+-- | 0.05 | 0.5 | 0.2 | 0.2 | 2.24 | 10 | 0.05 | 0.5 | 7.5 | 0.2 | 0.05 | 0.5 |
| 6 | -+-- --++ --+- | 0.05 | 2 | 0.05 | 0.05 | 0.56 | 2.5 | 0.2 | 0.5 | 7.5 | 0.05 | 0.2 | 0.5 |
| 7 | -+-- ++++ -+-+ | 0.05 | 2 | 0.05 | 0.05 | 2.24 | 10 | 0.2 | 0.5 | 7.5 | 0.2 | 0.05 | 2 |
| 8 | -+-+ ---- ++-- | 0.05 | 2 | 0.05 | 0.2 | 0.56 | 2.5 | 0.05 | 0.1 | 30 | 0.2 | 0.05 | 0.5 |
| 9 | -++- -+-- ++++ | 0.05 | 2 | 0.2 | 0.05 | 0.56 | 10 | 0.05 | 0.1 | 30 | 0.2 | 0.2 | 2 |
| 10 | -+++ +-+- +--- | 0.05 | 2 | 0.2 | 0.2 | 2.24 | 2.5 | 0.2 | 0.1 | 30 | 0.05 | 0.05 | 0.5 |
| 11 | +--- -++- -+-- | 0.2 | 0.5 | 0.05 | 0.05 | 0.56 | 10 | 0.2 | 0.1 | 7.5 | 0.2 | 0.05 | 0.5 |
| 12 | +--+ --++ ++-+ | 0.2 | 0.5 | 0.05 | 0.2 | 0.56 | 2.5 | 0.2 | 0.5 | 30 | 0.2 | 0.05 | 2 |

| Run | Pattern | | | | | | | | | | | | |
|---|---|---|---|---|---|---|---|---|---|---|---|---|---|
| 13 | +−−+ +++− +−+− | 0.2 | 0.5 | 0.05 | 0.2 | 2.24 | 10 | 0.2 | 0.1 | 30 | 0.05 | 0.2 | 0.5 |
| 14 | +−+− −−−+ +−−+ | 0.2 | 0.5 | 0.2 | 0.05 | 0.56 | 2.5 | 0.05 | 0.5 | 30 | 0.05 | 0.05 | 2 |
| 15 | +−+− +−−− −++− | 0.2 | 0.5 | 0.2 | 0.05 | 2.24 | 2.5 | 0.05 | 0.1 | 7.5 | 0.2 | 0.2 | 0.5 |
| 16 | ++−− +−−+ +++− | 0.2 | 2 | 0.05 | 0.05 | 2.24 | 2.5 | 0.05 | 0.5 | 30 | 0.2 | 0.2 | 0.5 |
| 17 | ++−+ −+−− −−++ | 0.2 | 2 | 0.05 | 0.2 | 0.56 | 10 | 0.05 | 0.1 | 7.5 | 0.05 | 0.2 | 2 |
| 18 | +++− +−+− −−−+ | 0.2 | 2 | 0.2 | 0.05 | 2.24 | 2.5 | 0.2 | 0.1 | 7.5 | 0.05 | 0.05 | 2 |
| 19 | ++++ −+−+ −−−− | 0.2 | 2 | 0.2 | 0.2 | 0.56 | 10 | 0.05 | 0.5 | 7.5 | 0.05 | 0.05 | 0.5 |
| 20 | ++++ ++++ ++++ | 0.2 | 2 | 0.2 | 0.2 | 2.24 | 10 | 0.2 | 0.5 | 30 | 0.2 | 0.2 | 2 |

*NTA = nitrilotriacetic acid

**Table 8. Absorbance read-outs indicating biomass concentration after 24 hours of fermentation in the Plackett-Burman experiment**

| Run | Pattern | Absorbance at 630 nm | | (a.u.) |
|---|---|---|---|---|
| | | Replicate 1 | Replicate 2 | Replicate 3 |
| 1 | ++−−+−−+ | 0.395 | 0.53 | 0.554 |
| 2 | −−−++−+−−++ | 0.572 | 0.575 | 0.569 |
| 3 | −−+−−++++−+− | 0.523 | 0.521 | 0.516 |
| 4 | −−++−−+−+++ | 0.718 | 0.757 | 0.652 |
| 5 | −−++++−+−+−− | 0.603 | 0.645 | 0.666 |

(continued)

| Run | Pattern | Absorbance at 630 nm | | (a.u.) |
| --- | --- | --- | --- | --- |
| | | Replicate 1 | Replicate 2 | Replicate 3 |
| 6 | -+-----++--+- | 0.594 | 0.59 | 0.607 |
| 7 | -+--++++-+-+ | 0.941 | 0.921 | 0.908 |
| 8 | -+-+----++-- | 0.795 | 0.752 | 0.76 |
| 9 | -++--+--++++ | 0.585 | 0.617 | 0.643 |
| 10 | -++++-+-+--- | 0.498 | 0.491 | 0.511 |
| 11 | +----++--+-- | 0.733 | 0.744 | 0.738 |
| 12 | +--+--++++-+ | 0.737 | 0.733 | 0.723 |
| 13 | +--++++-+-+- | 0.462 | 0.535 | 0.506 |
| 14 | +-+----++--+ | 0.506 | 0.457 | 0.472 |
| 15 | +-+-+----++- | 0.623 | 0.61 | 0.636 |
| 16 | ++-+--++++- | 0.796 | 0.813 | 0.827 |
| 17 | ++-+-+----++ | 0.463 | 0.503 | 0.437 |
| 18 | +++-+-+----+ | 0.477 | 0.36 | 0.428 |
| 19 | ++++-+-+---- | 0.41 | 0.38 | 0.408 |
| 20 | ++++++++++++ | 0.674 | 0.67 | 0.7 |

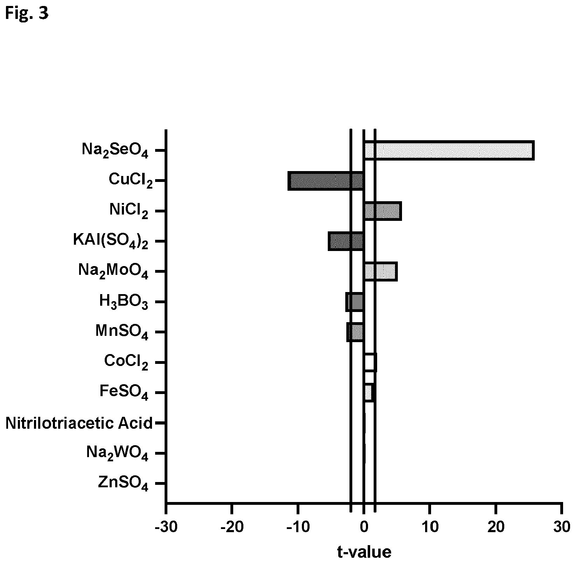

The results of the Plackett-Burman experiment are analysed with JMP (Figure 3). Components containing selenium, nickel, molybdenum and cobalt have a significantly positive effect on cell growth, while copper, aluminium, boric acid and manganese have a significantly negative effect on cell growth. The five components with the strongest effect ($Na_2SeO_4$, $CuCl_2$, $NiCl_2$, $KAl(SO_4)_2$ and $Na_2MoO_4$) are further tested using response surface methodology, in order to optimise their concentration in the growth medium.

The response surface experiment is performed as a central composite design with 3 centre points and an axial value of 2 is chosen, and the respective matrix is generated by JMP (Table 9). The high and low values are chosen based on the Plackett-Burman experiment, and the centre and axial points are calculated by JMP. $CuCl_2 \cdot 2H_2O$ and $KAl(SO_4)_2 \cdot 12H_2O$ have a negative effect, so the high level in RSM is chosen as the low level in Plackett-Burman, namely 0.05 mg/L for both. The low level is arbitrarily chosen as 0.01 mg/L for both. $Na_2MoO_4 \cdot 2H_2O$, $NiCl_2 \cdot 6H_2O$ and $Na_2SeO_4$ have a positive effect, so the low concentration in RSM is chosen as the high level in Plackett-Burman, as 0.2, 0.5 and 0.2 mg/L, respectively. The high levels are chosen arbitrarily as 0.6, 1.5 and 1 mg/L, respectively.

**Table 9. Response surface matrix used to optimise the concentrations of $KAl(SO_4)_2$, $CuCl_2$, $Na_2MoO_4$, $NiCl_2$ and $Na_2SeO_4$ for growth of *Moorella thermoacetica* on fructose**

| Run | Pattern | $KAl(SO_4)_2 \cdot 12H_2O$ (mg/L) | $CuCl_2 \cdot 2H_2O$ (mg/L) | $Na_2MoO_4 \cdot 2H_2O$ (mg/L) | $NiCl_2 \cdot 6H_2O$ (mg/L) | $Na_2SeO_4$ (mg/L) |
| --- | --- | --- | --- | --- | --- | --- |
| 1 | ----- | 0.01 | 0.01 | 0.20 | 0.50 | 0.20 |
| 2 | ---++ | 0.01 | 0.01 | 0.20 | 1.5 | 1.00 |
| 3 | --+-+ | 0.01 | 0.01 | 0.6 | 0.50 | 1.00 |
| 4 | --++- | 0.01 | 0.01 | 0.6 | 1.5 | 0.20 |
| 5 | -+--+ | 0.01 | 0.05 | 0.2 | 0.5 | 1.00 |
| 6 | -+-+- | 0.01 | 0.05 | 0.20 | 1.50 | 0.20 |
| 7 | -++-- | 0.01 | 0.05 | 0.60 | 0.50 | 0.20 |
| 8 | -++++ | 0.01 | 0.05 | 0.60 | 1.5 | 1.00 |

(continued)

| Run | Pattern | KAl(SO$_4$)$_2$·12H$_2$O (mg/L) | CuCl$_2$·2H$_2$O (mg/L) | Na$_2$MoO$_4$·2H$_2$O (mg/L) | NiCl$_2$·6H$_2$O (mg/L) | Na$_2$SeO$_4$ (mg/L) |
|---|---|---|---|---|---|---|
| 9 | +---+ | 0.05 | 0.01 | 0.2 | 0.50 | 1.00 |
| 10 | +--+- | 0.05 | 0.01 | 0.2 | 1.5 | 0.20 |
| 11 | +-+-- | 0.1 | 0.01 | 0.60 | 0.50 | 0.20 |
| 12 | +-+++ | 0.1 | 0.01 | 0.60 | 1.5 | 1.00 |
| 13 | ++--- | 0.1 | 0.05 | 0.20 | 0.5 | 0.20 |
| 14 | ++-++ | 0.1 | 0.05 | 0.20 | 1.5 | 1.00 |
| 15 | +++-+ | 0.1 | 0.05 | 0.60 | 0.5 | 1.00 |
| 16 | ++++- | 0.1 | 0.05 | 0.60 | 1.5 | 0.20 |
| 17 | a0000 | 0.0 | 0.03 | 0.40 | 1.0 | 0.60 |
| 18 | A0000 | 0.1 | 0.03 | 0.40 | 1.0 | 0.60 |
| 19 | 0a000 | 0.0 | 0.00 | 0.40 | 1.0 | 0.60 |
| 20 | 0A000 | 0.0 | 0.07 | 0.40 | 1.0 | 0.60 |
| 21 | 00a00 | 0.0 | 0.03 | 0.00 | 1.0 | 0.60 |
| 22 | 00A00 | 0.0 | 0.03 | 0.8 | 1.00 | 0.60 |
| 23 | 000a0 | 0.0 | 0.03 | 0.4 | 0.00 | 0.60 |
| 24 | 000A0 | 0.0 | 0.03 | 0.40 | 2.00 | 0.60 |
| 25 | 0000a | 0.0 | 0.03 | 0.40 | 1.0 | 0.00 |
| 26 | 0000A | 0.0 | 0.03 | 0.4 | 1.00 | 1.40 |
| 27 | 00000 | 0.0 | 0.03 | 0.4 | 1.0 | 0.60 |
| 28 | 00000 | 0.0 | 0.03 | 0.4 | 1.0 | 0.60 |
| 29 | 00000 | 0.0 | 0.03 | 0.4 | 1.0 | 0.60 |

The entire procedure of the response surface experiment is performed in an anaerobic chamber. 29 medium solutions are prepared according to the response surface matrix (Table 9), where the concentrations of Na$_2$SeO$_4$, CuCl$_2$·2H$_2$O, NiCl$_2$·6H$_2$O, KAl(SO$_4$)$_2$·12H$_2$O and Na$_2$MoO$_4$·2H$_2$O are varied by adding the stock solutions to a medium containing MES monohydrate (final concentration 20 g/L; pH 6.5), NH$_4$Cl (0.4 g/L), KH$_2$PO$_4$ (0.5 g/L), CaCl$_2$·2H$_2$O (0.05 g/L), MgCl$_2$·6H$_2$O (0.33 g/L), NaCl (0.4 g/L), fructose (5 g/L), yeast extract (0.5 g/L), vitamins (1 % v/v), CoCl$_2$·6H$_2$O (2 mg/L), H$_3$BO$_3$ (0.1 mg/L), FeSO$_4$·7H$_2$O (2.24 mg/L), MnSO$_4$·H$_2$O (10 mg/L), nitrilotriacetic acid (20 mg/L), Na$_2$WO$_4$·2H$_2$O (0.2 mg/L), ZnSO$_4$·7H$_2$O (2 mg/L), resazurin (0.001 g/L) and cysteine-HCl (1 mM). 490 $\mu$L of each solution is aliquoted to 3 wells of a 96-well deep-well plate, and 10 $\mu$L inoculum (OD$_{600}$ = 0.948) is added to all wells. The plates are placed in an anoxic box flushed with N$_2$/CO$_2$ (80/20), with a final pressure of 0.8 bars. The box is incubated at 60 °C without shaking. After 24 hours, the plates are removed from the incubator and each solution is transferred to a 96-well microtiter plate. The absorbance is read in a spectrophotometer at 630 nm (Table 10).

**Table 10. Absorbance read-outs indicating biomass concentration of the RSM experiment on KAl(SO$_4$)$_2$, CuCl$_2$, Na$_2$MoO$_4$, NiCl$_2$ and Na$_2$SeO$_4$**

| Runs | Pattern | Absorbance at 630 nm (a.u.) | | |
|---|---|---|---|---|
| | | Replicate 1 | Replicate 2 | Replicate 3 |
| 1 | ----- | 1.165 | 1.19 | 1.2 |
| 2 | ---++ | 1.176 | 1.234 | 1.222 |
| 3 | --+-+ | 1.293 | 1.304 | 1.264 |

(continued)

| Runs | Pattern | Absorbance at 630 nm (a.u.) | | |
|------|---------|-------------|-------------|-------------|
| | | Replicate 1 | Replicate 2 | Replicate 3 |
| 4 | --++- | 1.212 | 1.171 | 1.194 |
| 5 | -+--+ | 1.154 | 1.255 | 1.285 |
| 6 | -+-+- | 1.238 | 1.238 | 1.241 |
| 7 | -++-- | 1.223 | 1.238 | 1.2 |
| 8 | -++++ | 1.26 | 1.272 | 1.263 |
| 9 | +---+ | 1.162 | 1.26 | 1.312 |
| 10 | +--+- | 1.263 | 1.272 | 1.261 |
| 11 | +-+-- | 1.277 | 1.237 | 1.197 |
| 12 | +-+++ | 1.288 | 1.274 | 1.254 |
| 13 | ++--- | 1.131 | 1.203 | 1.234 |
| 14 | ++-++ | 1.262 | 1.285 | 1.271 |
| 15 | +++-+ | 1.288 | 1.299 | 1.305 |
| 16 | ++++- | 1.203 | 1.141 | 1.166 |
| 17 | a0000 | 1.148 | 1.265 | 1.313 |
| 18 | A0000 | 1.277 | 1.288 | 1.283 |
| 19 | 0a000 | 1.306 | 1.31 | 1.293 |
| 20 | 0A000 | 1.286 | 1.288 | 1.289 |
| 21 | 00a00 | 1.155 | 1.242 | 1.272 |
| 22 | 00A00 | 1.276 | 1.298 | 1.291 |
| 23 | 000a0 | 1.247 | 1.256 | 1.236 |
| 24 | 000A0 | 1.276 | 1.287 | 1.275 |
| 25 | 0000a | 0.317 | 0.504 | 0.474 |
| 26 | 0000A | 1.195 | 1.225 | 1.186 |
| 27 | 00000 | 1.239 | 1.226 | 1.211 |
| 28 | 00000 | 1.185 | 0.092 | 1.145 |
| 29 | 00000 | 1.095 | 1.095 | 1.102 |

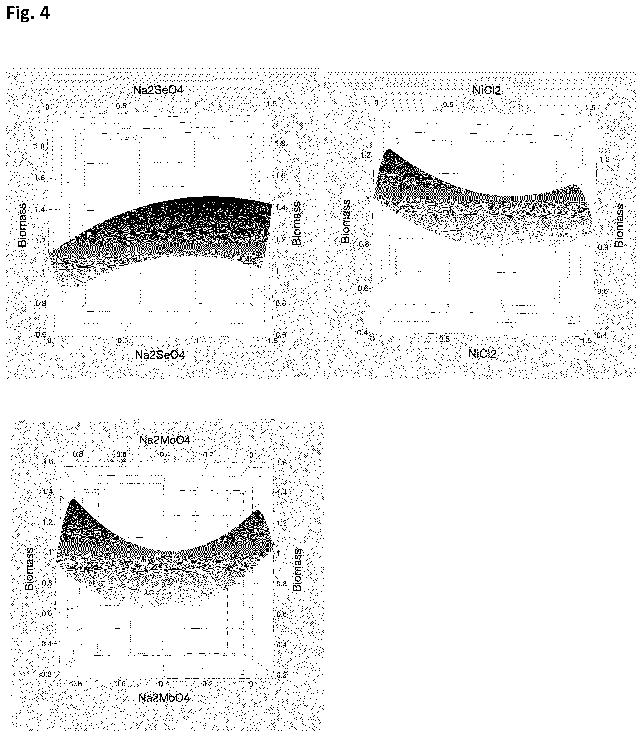

The results are analysed by JMP, which generates a second-degree polynomial equation (Equation 2) and the corresponding response surface plots (Figure 4). The analysis shows that, within the boundaries tested, biomass concentration increases with increased $Na_2SeO_4$ concentration up to a critical level, after which it starts decreasing. At the same time, the highest biomass concentration is achieved at the highest $Na_2MoO_4$ concentration tested. With respect to $NiCl_2$, the model suggests that the highest biomass concentration is achieved when using a nickel concentration close to the two boundaries tested.

$$1.01783470999122 + -0.000929736362079477 * ((KAl(SO_4)_2 - 0.03) / 0.02) +$$

$$-0.0123591196301995 * ((CuCl_2 - 0.03) / 0.02) + 0.00973611111111112 * ((Na_2MoO_4$$

$$-0.4) / 0.2) + 0.00254166666666666 * ((NiCl_2 - 1) / 0.5) + 0.0833694065482988 * ((
$$

$$Na_2SeO_4 - 0.6) / 0.4) + (KAl(SO_4)_2 - 0.03) / 0.02 * (CuCl_2 - 0.03) / 0.02 *$$

$$-0.0106458333333333 + (KAl(SO_4)_2 - 0.03) / 0.02 * (Na_2MoO_4 - 0.4) / 0.2 *$$

$$-0.00589583333333332 + (CuCl_2 - 0.03) / 0.02 * (Na_2MoO_4 - 0.4) / 0.2 *$$

$$-0.00389583333333333 + (KAl(SO_4)_2 - 0.03) / 0.02 * (NiCl_2 - 1) / 0.5 *$$

$$0.00177083333333332 + (CuCl_2 - 0.03) / 0.02 * (NiCl_2 - 1) / 0.5 *$$

$$0.00135416666666668 + (Na_2MoO_4 - 0.4) / 0.2 * (NiCl_2 - 1) / 0.5 *$$

$$-0.0174791666666667 + (KAl(SO_4)_2 - 0.03) / 0.02 * (Na_2SeO_4 - 0.6) / 0.4 *$$

$$0.00422916666666669 + (CuCl_2 - 0.03) / 0.02 * (Na_2SeO_4 - 0.6) / 0.4 *$$

$$0.00706249999999999 + (Na_2MoO_4 - 0.4) / 0.2 * (Na_2SeO_4 - 0.6) / 0.4 * 0.0138125 + (
$$

$$NiCl_2 - 1) / 0.5 * (Na_2SeO_4 - 0.6) / 0.4 * -0.0046875 + (KAl(SO_4)_2 - 0.03) / 0.02$$

$$* (KAl(SO_4)_2 - 0.03) / 0.02 * 0.071733898175278 + (CuCl_2 - 0.03) / 0.02 * (CuCl_2$$

$$- 0.03) / 0.02 * 0.0817094897534409 + (Na_2MoO_4 - 0.4) / 0.2 * (Na_2MoO_4 - 0.4) / 0.2$$

$$* 0.0576734726195067 + (NiCl_2 - 1) / 0.5 * (NiCl_2 - 1) / 0.5 * 0.0594651392861734$$

$$+ (Na_2SeO_4 - 0.6) / 0.4 * (Na_2SeO_4 - 0.6) / 0.4 * -0.048660176726902$$

**Equation 2. Second degree polynomial equation resulted from the RSM experiment on KAl(SO$_4$)$_2$, CuCl$_2$, Na$_2$MoO$_4$, NiCl$_2$ and Na$_2$SeO$_4$**

**Example 3.**

This example describes the improved biomass production of *Moorella thermoacetica* in a growth medium containing fructose as carbon source and novel concentrations of certain nutrients.

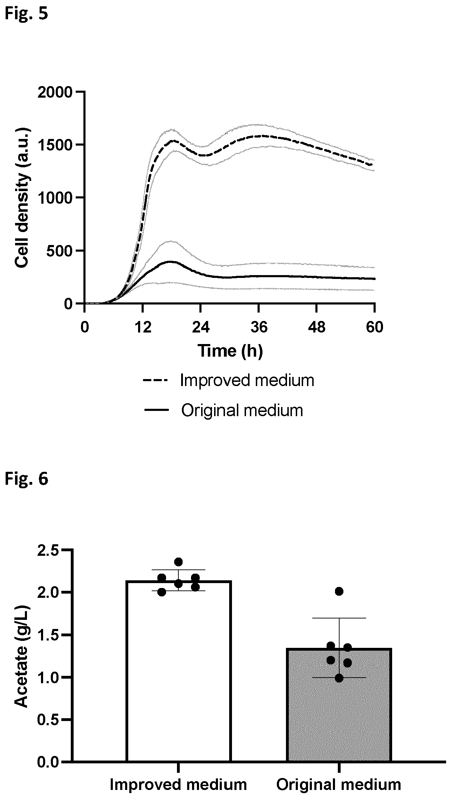

The procedure is performed in an anaerobic chamber to ensure an anoxic environment. As per Examples 1 and 2, sterile and anaerobic stock solutions of each component are prepared. Two media, termed "original" and "improved" media, are prepared by mixing stock solutions to reach the concentrations shown in Table 11. Then, 49 mL of each medium are aliquoted into three 125 mL serum bottles and inoculated with a growing culture of *Moorella thermoacetica* (OD$_{600}$= 0.86) to reach an initial OD$_{600}$ of 0.02. The vials are incubated at 60 °C while stirring at 200 rpm using magnetic stirrers. The biomass concentration is monitored using a real-time cell density monitoring device. The growth pattern in the two media is presented in Figure 5, which shows that the improved medium supports faster growth and higher biomass densities. According to end-point manual $OD_{600}$ measurements, biomass concentration is 63 % higher in the improved medium than the original medium after 63 hours of fermentation. At the same time, Figure 6 shows that the improved medium increased acetate production by 60 % on average.

**Table 11. Concentrations of components in the "original" and "improved" media using fructose as carbon source for fermentation with *Moorella thermoacetica***

| Component | Concentration in "original medium" | Concentration in "improved medium" |
|---|---|---|
| Fructose (g/L) | 5 | 5 |
| MES monohydrate (g/L) | 20 | 20 |
| Resazurin (g/L) | 0.001 | 0.001 |
| Cysteine-HCl (mM) | 1 | 1 |
| Yeast extract (g/L) | 0.5 | 0.5 |
| $NH_4Cl$ (g/L) | 0.4 | 0.4 |
| $MgCl_2 \cdot 6H_2O$ (g/L) | 0.33 | 0.05 |
| $CaCl_2 \cdot 2H_2O$ (g/L) | 0.05 | 0.05 |
| $KH_2PO_4$ (g/L) | 0.5 | 0.5 |
| NaCl (g/L) | 0.4 | 0.4 |
| Biotin (mg/L) | 0.02 | 0.12 |
| Folic acid (mg/L) | 0.02 | 0.02 |
| Pyridoxine-HCl (mg/L) | 0.1 | 0.1 |
| Thiamine-HCl (mg/L) | 0.05 | 0.1 |
| Riboflavin (mg/L) | 0.05 | 0.05 |
| Nicotinic acid (mg/L) | 0.05 | 0.05 |
| Ca-D-Pantothenate (mg/L) | 0.05 | 0.01 |
| Vit B12 (mg/L) | 0.005 | 0.0005 |
| p-aminobenzoic acid (mg/L) | 0.05 | 0.05 |
| Thioctic acid (mg/L) | 0.05 | 0.3 |
| $CoCl_2 \cdot 6H_2O$ (mg/L) | 2 | 2 |
| $CuCl_2 \cdot 2H_2O$ (mg/L) | 0.2 | 0.01 |
| $FeSO_4 \cdot 7H_2O$ (mg/L) | 5.6 | 5.6 |
| $MnSO_4 \cdot H_2O$ (mg/L) | 10 | 7.5 |
| $Na_2MoO_4 \cdot 2H_2O$ (mg/L) | 0.2 | 0.6 |
| $NiCl_2 \cdot 6H_2O$ (mg/L) | 0.2 | 1.5 |
| Nitrilotriacetic acid (mg/L) | 20 | 20 |
| $Na_2SeO_4$ (mg/L) | 0.2 | 1 |
| $Na_2WO_4 \cdot 2H_2O$ (mg/L) | 0.2 | 0.2 |
| $ZnSO_4 \cdot 7H_2O$ (mg/L) | 2 | 2 |

**Example 4**

This example describes the optimisation of medium composition with respect to five macronutrients specifically

for growth on $CO_2$ and $H_2$ using Plackett-Burman design and response surface methodology.

The following stock solutions are prepared and sterilised: yeast extract (100 g/L), $NH_4Cl$ (40 g/L), $MgCl_2 \cdot 6H_2O$ (20 g/L), $CaCl_2 \cdot 2H_2O$ (10 g/L), $KH_2PO_4$ (20 g/L), NaCl (20 g/L), MES monohydrate (200 g/L, pH 6.5), cysteine-HCl (100 mM) and resazurin (0.01 g/L). The trace elements solution contains nitrilotriacetic acid (1 g/L; pH 6 adjusted with KOH), $MnSO_4 \cdot H_2O$ (20 g/L), $Fe(SO_4) \cdot 7H_2O$ (14 g/L), $CoCl_2 \cdot 6H_2O$ (10 g/L), $ZnSO_4 \cdot 7H_2O$ (4 g/L), $CuCl_2 \cdot 2H_2O$ (1 g/L), $NiCl_2 \cdot 6H_2O$ (1 g/L), $Na_2MoO_4 \cdot 2H_2O$ (1 g/L), $Na_2SeO_4$ (1 g/L) and $Na_2WO_4 \cdot 2H_2O$ (1 g/L). The vitamin solution is filter-sterilised and contains biotin (2 mg/L), folic acid (2 mg/L), pyridoxine hydrochloride (10 mg/L), thiamine-HCl (5 mg/L), riboflavin (5 mg/L), nicotinic acid (5 mg/L), calcium-D-(+)-pantothenate (5 mg/L), Vitamin B12 (0.5 mg/L), p-aminobenzoic acid (5 mg/L), thioctic acid (5 mg/L).

Two levels are chosen for each factor tested (Table 12). The Placket-Burman matrix, describing the final concentration of each factor in the 20 experiments performed, is generated with the statistical program JMP (Table 13). The experimental procedure is performed in an anaerobic chamber to guarantee an anoxic environment. Anoxic and sterile stock solutions are mixed to reach final concentrations as indicated in Table 13, along with MES monohydrate (final concentration 20 g/L; pH 6.5), yeast extract (0.5 g/L), vitamin solution (1 % v/v), trace elements solution (1 % v/v) resazurin (0.001 g/L) and cysteine-HCl (1 mM). 490 $\mu$L of each medium mix is aliquoted to 3 wells of a 96-well deep-well plate, and 10 $\mu$L inoculum is added to all wells. The plates are placed in an anoxic box, flushed with $H_2/CO_2$ (80/20) for 10 minutes and, with a final pressure of 0.6 bars, incubated at 60 °C without shaking. After 24 hours, the plates are removed from the incubator and the culture of each well is transferred to a 96-well microtiter plate. The absorbance is read in a spectrophotometer at 630 nm (Table 14).

**Table 12. The two levels selected for each factor tested using Plackett-Burman design and $CO_2$ as carbon source**

| Level | $NH_4Cl$ (g/L) | $MgCl_2 \cdot 6H_2O$ (g/L) | $CaCl_2 \cdot 2H_2O$ (g/L) | $KH_2PO_4$ (g/L) | NaCl (g/L) |
|---|---|---|---|---|---|
| Low (-) | 0.2 | 0.05 | 0.01 | 0.1 | 0.1 |
| High (+) | 2 | 0.5 | 0.1 | 1 | 1 |

**Table 13. Plackett-Burman matrix used to examine which of the factors tested have a significant effect on the growth of *Moorella thermoacetica* on $CO_2$**

| Runs | Pattern | $NH_4Cl$ (g/L) | $MgCl_2 \cdot 6H_2O$ (g/L) | $CaCl_2 \cdot 2H_2O$ (g/L) | $KH_2PO_4$ (g/L) | NaCl (g/L) |
|---|---|---|---|---|---|---|
| 1 | +++++ | 2.0 | 0.50 | 0.10 | 1.0 | 1.0 |
| 2 | -+-++ | 0.2 | 0.50 | 0.01 | 1.0 | 1.0 |
| 3 | --+-+ | 0.2 | 0.05 | 0.10 | 0.1 | 1.0 |
| 4 | +--+- | 2.0 | 0.05 | 0.01 | 1.0 | 0.1 |
| 5 | -+--+ | 0.2 | 0.50 | 0.01 | 0.1 | 1.0 |
| 6 | --+-- | 0.2 | 0.05 | 0.10 | 0.1 | 0.1 |
| 7 | ---+- | 0.2 | 0.05 | 0.01 | 1.0 | 0.1 |
| 8 | +---+ | 2.0 | 0.05 | 0.01 | 0.1 | 1.0 |
| 9 | ++--- | 2.0 | 0.50 | 0.01 | 0.1 | 0.1 |
| 10 | +++-- | 2.0 | 0.50 | 0.10 | 0.1 | 0.1 |
| 11 | -+++- | 0.2 | 0.50 | 0.10 | 1.0 | 0.1 |
| 12 | +-+++ | 2.0 | 0.05 | 0.10 | 1.0 | 1.0 |
| 13 | 00000 | 1.1 | 0.28 | 0.06 | 0.6 | 0.6 |
| 14 | 00000 | 1.1 | 0.28 | 0.06 | 0.6 | 0.6 |
| 15 | 00000 | 1.1 | 0.28 | 0.06 | 0.6 | 0.6 |

**Table 14. Absorbance read-outs indicating biomass concentration after 24 hours of fermentation in the Plackett-Burman experiment**

| Runs | Pattern | Absorbance at 630 nm (a.u.) | | |
|---|---|---|---|---|
| | | Replicate 1 | Replicate 2 | Replicate 3 |
| 1 | +++++ | 0.160 | 0.160 | 0.159 |
| 2 | -+-++ | 0.163 | 0.179 | 0.166 |
| 3 | --+-+ | 0.169 | 0.168 | 0.165 |
| 4 | +--+- | 0.138 | 0.150 | 0.140 |
| 5 | -+--+ | 0.182 | 0.174 | 0.167 |
| 6 | --+-- | 0.167 | 0.166 | 0.162 |
| 7 | ---+- | 0.152 | 0.145 | 0.147 |
| 8 | +---+ | 0.136 | 0.138 | 0.136 |
| 9 | ++--- | 0.164 | 0.159 | 0.154 |
| 10 | +++-- | 0.156 | 0.148 | 0.157 |
| 11 | -+++- | 0.157 | 0.150 | 0.149 |
| 12 | +-+++ | 0.133 | 0.134 | 0.134 |
| 13 | 00000 | 0.174 | 0.169 | 0.168 |
| 14 | 00000 | 0.164 | 0.161 | 0.157 |
| 15 | 00000 | 0.164 | 0.158 | 0.160 |

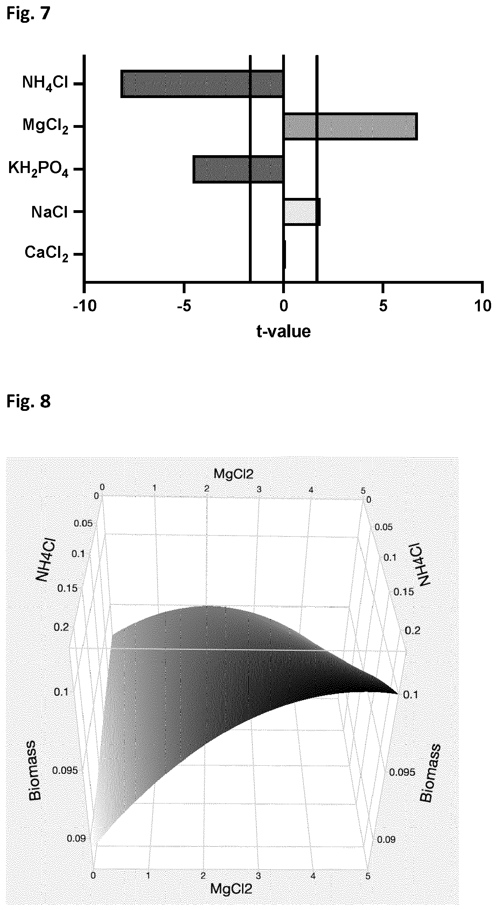

The results of the Plackett-Burman experiment are analysed with JMP (Figure 7). The analysis shows that, when $CO_2$ is used as carbon source, $NH_4Cl$ and $KH_2PO_4$ have a significantly negative effect on growth, while $MgCl_2$ and NaCl have a significantly positive effect. $CaCl_2$ does not have a significant effect. $NH_4Cl$, $KH_2PO_4$, $MgCl_2$ and NaCl are further tested using response surface methodology.

The response surface experiment is performed as a central composite design with 3 centre points and an axial value of 1 is chosen, and the respective matrix is generated by JMP (Table 15). The high and low values are chosen based on the Plackett-Burman experiment, and the centre and axial points are calculated by JMP. $NH_4Cl$ and $KH_2PO_4$ have a negative effect in Plackett-Burman, so the high concentration in RSM is the same as the low concentration in Plackett-Burman, 0.2 and 0.1 g/L, respectively. The low value is chosen as 0.02 and 0.01 g/L, respectively. Since $MgCl_2$ and NaCl have a positive effect on growth, the low value in RSM is chosen as 0.5 and 1 g/L, respectively, and the high value as 5 g/L for both compounds.

**Table 25. Response surface matrix used to optimise the concentrations of $NH_4Cl$, $MgCl_2$, $KH_2PO_4$ and NaCl for growth of *Moorella thermoacetica* on $CO_2$**

| Runs | Pattern | $NH_4Cl$ (g/L) | $MgCl_2 \cdot 6H_2O$ (g/L) | $KH_2PO_4$ (g/L) | NaCl (g/L) |
|---|---|---|---|---|---|
| 1 | ---- | 0.02 | 0.50 | 0.01 | 1.0 |
| 2 | ---+ | 0.02 | 0.50 | 0.01 | 5 |
| 3 | --+- | 0.02 | 0.50 | 0.10 | 1 |
| 4 | --++ | 0.02 | 0.50 | 0.10 | 5 |
| 5 | -+-- | 0.02 | 5.00 | 0.01 | 1 |
| 6 | -+-+ | 0.02 | 5.00 | 0.01 | 5 |
| 7 | -++- | 0.02 | 5.00 | 0.10 | 1 |
| 8 | -+++ | 0.02 | 5.00 | 0.10 | 5 |
| 9 | +--- | 0.20 | 0.50 | 0.01 | 1 |

(continued)

| Runs | Pattern | NH₄Cl (g/L) | MgCl₂·6H₂O (g/L) | KH₂PO₄ (g/L) | NaCl (g/L) |
|------|---------|-------------|-------------------|--------------|------------|
| 10 | +--+ | 0.20 | 0.50 | 0.01 | 5 |
| 11 | +-+- | 0.20 | 0.50 | 0.10 | 1 |
| 12 | +-++ | 0.20 | 0.50 | 0.10 | 5 |
| 13 | ++-- | 0.20 | 5.00 | 0.01 | 1 |
| 14 | ++-+ | 0.20 | 5.00 | 0.01 | 5 |
| 15 | +++- | 0.20 | 5.00 | 0.10 | 1 |
| 16 | ++++ | 0.20 | 5.00 | 0.10 | 5 |
| 17 | a000 | 0.02 | 2.75 | 0.06 | 3 |
| 18 | A000 | 0.20 | 2.75 | 0.06 | 3 |
| 19 | 0a00 | 0.11 | 0.50 | 0.06 | 3 |
| 20 | 0A00 | 0.11 | 5.00 | 0.06 | 3 |
| 21 | 00a0 | 0.11 | 2.75 | 0.01 | 3 |
| 22 | 00A0 | 0.11 | 2.75 | 0.10 | 3 |
| 23 | 000a | 0.11 | 2.75 | 0.06 | 1 |
| 24 | 000A | 0.11 | 2.75 | 0.06 | 5 |
| 25 | 0000 | 0.11 | 2.75 | 0.06 | 3 |
| 26 | 0000 | 0.11 | 2.75 | 0.06 | 3 |
| 27 | 0000 | 0.11 | 2.75 | 0.06 | 3 |

The entire procedure of the response surface experiment is performed in an anaerobic chamber. 27 medium solutions are prepared according to the response surface matrix (Table 15), where the concentrations of $NH_4Cl$, $KH_2PO_4$, $MgCl_2 \cdot 6H_2O$ and NaCl are varied by adding the stock solutions to a medium containing MES monohydrate (final concentration 20 g/L; pH 6.5), $CaCl_2 \cdot 2H_2O$ (0.05 g/L), yeast extract (0.5 g/L), vitamins (1 % v/v), trace elements solution (1 % v/v), resazurin (0.001 g/L) and cysteine-HCl (1 mM). 490 μL of each solution is aliquoted to 3 wells of a 96-well deep-well plate, and 10 μL inoculum is added to all wells. The plates are placed in an anoxic box flushed with $H_2/CO_2$ (80/20) for 10 minutes, with a final pressure of 0.8 bars. The box is incubated at 60 °C without shaking. After 24 hours, the plates are removed from the incubator and each solution is transferred to a 96-well microtiter plate. The absorbance is read in a spectrophotometer at 630 nm (Table 16).

**Table 16. Absorbance read-outs indicating biomass concentration of the RSM experiment on NH₄Cl, MgCl₂, KH₂PO₄ and NaCl**

| Runs | Pattern | Absorbance at 630 nm (a.u.) | | |
|------|---------|-------------|-------------|-------------|
| | | Replicate 1 | Replicate 2 | Replicate 3 |
| 1 | ---- | 0.104 | 0.105 | 0.098 |
| 2 | ---+ | 0.085 | 0.089 | 0.089 |
| 3 | --+- | 0.097 | 0.098 | 0.099 |
| 4 | --++ | 0.089 | 0.084 | 0.084 |
| 5 | -+-- | 0.099 | 0.102 | 0.1 |
| 6 | -+-+ | 0.089 | 0.088 | 0.087 |
| 7 | -++- | 0.098 | 0.094 | 0.093 |
| 8 | -+++ | 0.085 | 0.085 | 0.083 |

(continued)

| Runs | Pattern | Absorbance at 630 nm (a.u.) | | |
|------|---------|-------------|-------------|-------------|
|      |         | Replicate 1 | Replicate 2 | Replicate 3 |
| 9 | +--- | 0.098 | 0.1 | 0.098 |
| 10 | +--+ | 0.095 | 0.079 | 0.092 |
| 11 | +-+- | 0.092 | 0.088 | 0.101 |
| 12 | +-++ | 0.089 | 0.088 | 0.086 |
| 13 | ++-- | 0.116 | 0.115 | 0.114 |
| 14 | ++-+ | 0.106 | 0.099 | 0.101 |
| 15 | +++- | 0.096 | 0.097 | 0.096 |
| 16 | ++++ | 0.091 | 0.086 | 0.086 |
| 17 | a000 | 0.098 | 0.098 | 0.095 |
| 18 | A000 | 0.098 | 0.097 | 0.093 |
| 19 | 0a00 | 0.097 | 0.095 | 0.096 |
| 20 | 0A00 | 0.095 | 0.095 | 0.086 |
| 21 | 00a0 | 0.104 | 0.101 | 0.1 |
| 22 | 00A0 | 0.098 | 0.095 | 0.101 |
| 23 | 000a | 0.101 | 0.1 | 0.103 |
| 24 | 000A | 0.089 | 0.085 | 0.087 |
| 26 | 0000 | 0.096 | 0.099 | 0.095 |
| 27 | 0000 | 0.1 | 0.098 | 0.097 |

The results are analysed by JMP, which generates a second-degree polynomial equation (Equation 3). The corresponding response surface plot showing the $MgCl_2$ trend is presented in Figure 7. When the carbon source is $CO_2$, biomass concentration increases with $MgCl_2$ concentration until a critical point is reached, after which the biomass concentration plateaus even when further increasing the $MgCl_2$ concentration.

$$0.0969428571428572 + 0.00151851851851852 * ((NH_4Cl - 0.11) / 0.09)$$

$$+0.00124074074074074 * ((MgCl_2 - 2.75) / 2.25) + -0.00322222222222222 * ((KH_2PO_4$$

$$-0.055) / 0.045) + -0.00566666666666667 * ((NaCl - 3) / 2) + (NH_4Cl - 0.11) / 0.09$$

$$* (MgCl_2 - 2.75) / 2.25 * 0.00239583333333333 + (NH_4Cl - 0.11) / 0.09 * (KH_2PO_4$$

$$-0.055) / 0.045 * -0.00147916666666667 + (MgCl_2 - 2.75) / 2.25 * (KH_2PO_4 - 0.055) /$$

$$0.045 * -0.00185416666666667 + (NH_4Cl - 0.11) / 0.09 * (NaCl - 3) / 2 *$$

$$0.000770833333333332 + (MgCl_2 - 2.75) / 2.25 * (NaCl - 3) / 2 *$$

$$-0.000104166666666667 + (KH_2PO_4 - 0.055) / 0.045 * (NaCl - 3) / 2 *$$

$$0.000770833333333333 + (NH_4Cl - 0.11) / 0.09 * (NH_4Cl - 0.11) / 0.09 *$$

$$-0.000257142857142861 + (MgCl_2 - 2.75) / 2.25 * (MgCl_2 - 2.75) / 2.25 *$$

$$-0.00275714285714286 + (KH_2PO_4 - 0.055) / 0.045 * (KH_2PO_4 - 0.055) / 0.045 *$$

$$0.00307619047619048 + (NaCl - 3) / 2 * (NaCl - 3) / 2 * -0.00259047619047619$$

**Equation 3. Second degree polynomial equation resulted from the RSM experiment on NH$_4$Cl, MgCl$_2$, KH$_2$PO$_4$ and NaCl**

**Example 5**

This experiment describes the statistical optimisation of selected trace elements and vitamins for improved growth of *Moorella thermoacetica* on $H_2/CO_2$. Plackett-Burman design is used to identify which components have a significant effect on cell growth and response surface methodology (RSM) is used to determine improved concentrations of those components. The factors tested in this experiment are the following 14 components: biotin, thiamine-HCl, Ca-D-(+)-pantothenate, vitamin B12, nicotinic acid, thioctic acid, $CoCl_2$, $CuCl_2$, $FeSO_4$, $MnSO_4$, $NiCl_2$, $Na_2SeO_4$, $Na_2WO_4$, $Na_2MoO_4$.

The following stock solutions are prepared and sterilised: yeast extract (100 g/L), $NH_4Cl$ (40 g/L), $MgCl_2 \cdot 6H_2O$ (20 g/L), $CaCl_2 \cdot 2H_2O$ (10 g/L), $KH_2PO_4$ (20 g/L), NaCl (20 g/L), MES monohydrate (200 g/L, pH 6.5), cysteine-HCl (100 mM), resazurin (0.01 g/L), nitrilotriacetic acid (1 g/L; pH 6 adjusted with KOH), $MnSO_4 \cdot H_2O$ (20 g/L), $Fe(SO_4) \cdot 7H_2O$ (14 g/L), $CoCl_2 \cdot 6H_2O$ (10 g/L), $ZnSO_4 \cdot 7H_2O$ (4 g/L), $CuCl_2 \cdot 2H_2O$ (1 g/L), $NiCl_2 \cdot 6H_2O$ (1 g/L), $Na_2MoO_4 \cdot 2H_2O$ (1 g/L), $Na_2SeO_4$ (1 g/L), $Na_2WO_4 \cdot 2H_2O$ (1 g/L), biotin (0.5 g/L), folic acid (1 g/L), pyridoxine hydrochloride (1 g/L), thiamine-HCl (1 g/L), riboflavin (0.1 g/L), nicotinic acid (1 g/L), calcium-D-(+)-pantothenate (1 g/L), Vitamin B12 (1 g/L), p-aminobenzoic acid (1 g/L) and thioctic acid (1 g/L).

The initial screening is performed on two levels (Table 17). The Placket-Burman matrix, describing the final concentration of each factor in the 23 experiments performed, is generated with the statistical program JMP (Table 18). The experimental procedure is performed in an anaerobic chamber to guarantee an anoxic environment. Anoxic and sterile stock solutions are mixed to reach final concentrations as indicated in Table 18, along with MES monohydrate (final concentration 20 g/L; pH 6.5), yeast extract (0.5 g/L), $NH_4Cl$ (0.4 g/L), $MgCl_2 \cdot 6H_2O$ (0.33 g/L), $CaCl_2 \cdot 2H_2O$ (0.05 g/L), $KH_2PO_4$ (0.5 g/L), NaCl (0.4 g/L), folic acid (0.02 mg/L), pyridoxine-HCl (0.1 mg/L), riboflavin (0.05 mg/L), p-aminobenzoic acid (0.05 mg/L), $ZnSO_4 \cdot 7H_2O$ (2 mg/L), nitrilotriacetic acid (20 mg/L), resazurin (0.001 g/L) and cysteine-HCl (1 mM). 490 $\mu$L of each medium mix is aliquoted to 3 wells of a 96-well deep-well plate, and 10 $\mu$L inoculum is added to all wells. The plates are placed in an anoxic box, flushed with $H_2/CO_2$ (80/20) for 10 minutes and, with a final pressure of 0.8 bars, incubated at 60 °C without shaking. After 24 hours, the plates are removed from the incubator and the culture of each well is transferred to a 96-well microtiter plate. The absorbance is read in a spectrophotometer at 630 nm (Table 19).

Table 17. The two levels selected for each factor tested using Plackett-Burman design and $CO_2$ as carbon source

| Level | Biotin (mg/L) | Thiamine-HCl (mg/L) | Ca-D-pantothenate (mg/L) | Vitamin B12 (mg/L) | Nicotinic acid (mg/L) | Thioctic acid (mg/L) | $MnSO_4 \cdot H_2O$ (mg/L) | $CuCl_2 \cdot 2H_2O$ (mg/L) | $NiCl_2 \cdot 6H_2O$ (mg/L) | $Na_2MoO_4 \cdot 2H_2O$ (mg/L) | $Na_2SeO_4$ (mg/L) | $Na_2WO_4 \cdot 2H_2O$ (mg/L) | $CoCl_2 \cdot 6H_2O$ (mg/L) | $FeSO_4 \cdot 7H_2O$ (mg/L) |
|---|---|---|---|---|---|---|---|---|---|---|---|---|---|---|
| Low (-) | 0.02 | 0.05 | 0.05 | 0.005 | 0.05 | 0.05 | 5 | 0.05 | 0.2 | 0.2 | 0.2 | 0.2 | 2 | 5.6 |
| High (+) | 0.12 | 0.1 | 0.1 | 0.01 | 0.1 | 0.3 | 10 | 0.2 | 1.5 | 0.6 | 1 | 0.4 | 4 | 11.2 |

Table 18. Plackett-Burman matrix used to examine which of the factors tested have a significant effect on the growth of *Moorella thermoacetica* on $CO_2$.

| Run | Pattern | Biotin (mg/L) | Thiamine-HCl (mg/L) | Ca-D-pantothenate (mg/L) | Vitamin B12 (mg/L) | Nicotinic acid (mg/L) | Thioctic acid (mg/L) | $MnSO_4 \cdot H_2O$ (mg/L) | $CuCl_2 \cdot 2H_2O$ (mg/L) | $NiCl_2 \cdot 6H_2O$ (mg/L) | $Na_2MoO_4 \cdot 2H_2O$ (mg/L) | $Na_2SeO_4$ (mg/L) | $Na_2WO_4 \cdot 2H_2O$ (mg/L) | $CoCl_2 \cdot 6H_2O$ (mg/L) | $FeSO_4 \cdot 7H_2O$ (mg/L) |
|---|---|---|---|---|---|---|---|---|---|---|---|---|---|---|---|
| 1 | ----+ +--+- -+++ | 0.02 | 0.05 | 0.05 | 0.005 | 0.10 | 0.30 | 5 | 0.05 | 1.5 | 0.2 | 0.2 | 0.4 | 4 | 11.2 |
| 2 | ---++ --+-- ++++ | 0.02 | 0.05 | 0.05 | 0.010 | 0.10 | 0.05 | 5 | 0.20 | 0.2 | 0.2 | 1.0 | 0.4 | 4 | 11.2 |
| 3 | --+-- ++++- +-+- | 0.02 | 0.05 | 0.10 | 0.005 | 0.05 | 0.30 | 10 | 0.20 | 1.5 | 0.2 | 1.0 | 0.2 | 4 | 5.6 |

| | | | | | | | | | | | | | | | |
|---|---|---|---|---|---|---|---|---|---|---|---|---|---|---|---|
| 4 | --+-<br>-+--+<br>+++- | 0.02 | 0.05 | 0.10 | 0.010 | 0.05 | 0.05 | 10 | 0.05 | 0.2 | 0.6 | 1.0 | 0.4 | 4 | 5.6 |
| 5 | --+++<br>+-+-+<br>---- | 0.02 | 0.05 | 0.10 | 0.010 | 0.10 | 0.30 | 5 | 0.20 | 0.2 | 0.6 | 0.2 | 0.2 | 2 | 5.6 |
| 6 | -+---<br>-++--<br>+--+ | 0.02 | 0.10 | 0.05 | 0.005 | 0.05 | 0.05 | 10 | 0.20 | 0.2 | 0.2 | 1.0 | 0.2 | 2 | 11.2 |
| 7 | -+--+<br>+++-+<br>-+-- | 0.02 | 0.10 | 0.05 | 0.005 | 0.10 | 0.30 | 10 | 0.20 | 0.2 | 0.6 | 0.2 | 0.4 | 2 | 5.6 |
| 8 | -+-+-<br>---++<br>--+- | 0.02 | 0.10 | 0.05 | 0.010 | 0.05 | 0.05 | 5 | 0.05 | 1.5 | 0.6 | 0.2 | 0.2 | 4 | 5.6 |
| 9 | -++--<br>+--++<br>++-+ | 0.02 | 0.10 | 0.10 | 0.005 | 0.05 | 0.30 | 5 | 0.05 | 1.5 | 0.6 | 1.0 | 0.4 | 2 | 11.2 |
| 10 | -++++<br>-+-+-<br>---+ | 0.02 | 0.10 | 0.10 | 0.010 | 0.10 | 0.05 | 10 | 0.05 | 1.5 | 0.2 | 0.2 | 0.2 | 2 | 11.2 |
| 11 | 00000<br>00000<br>0000 | 0.07 | 0.075 | 0.075 | 0.0075 | 0.075 | 0.175 | 7.5 | 0.125 | 0.85 | 0.4 | 0.6 | 0.3 | 3 | 8.4 |
| 12 | 00000<br>00000<br>0000 | 0.07 | 0.075 | 0.075 | 0.0075 | 0.075 | 0.175 | 7.5 | 0.125 | 0.85 | 0.4 | 0.6 | 0.3 | 3 | 8.4 |
| 13 | 00000<br>00000<br>0000 | 0.07 | 0.075 | 0.075 | 0.0075 | 0.075 | 0.175 | 7.5 | 0.125 | 0.85 | 0.4 | 0.6 | 0.3 | 3 | 8.4 |

| Run | Pattern | | | | | | | | | | | | | | |
|---|---|---|---|---|---|---|---|---|---|---|---|---|---|---|---|
| 14 | +---- ++--+ --++ | 0.12 | 0.05 | 0.05 | 0.005 | 0.05 | 0.30 | 10 | 0.05 | 0.2 | 0.6 | 0.2 | 0.2 | 4 | 11.2 |
| 15 | +--+- -++++ -+-+ | 0.12 | 0.05 | 0.05 | 0.010 | 0.05 | 0.05 | 10 | 0.20 | 1.5 | 0.6 | 0.2 | 0.4 | 2 | 11.2 |
| 16 | +--++ ++-+- +--- | 0.12 | 0.05 | 0.05 | 0.010 | 0.10 | 0.30 | 10 | 0.05 | 1.5 | 0.2 | 1.0 | 0.2 | 2 | 5.6 |
| 17 | +-+-- --++- -+-- | 0.12 | 0.05 | 0.10 | 0.005 | 0.05 | 0.05 | 5 | 0.20 | 1.5 | 0.2 | 0.2 | 0.4 | 2 | 5.6 |
| 18 | +-+-+ ----+ +--+ | 0.12 | 0.05 | 0.10 | 0.005 | 0.10 | 0.05 | 5 | 0.05 | 0.2 | 0.6 | 1.0 | 0.2 | 2 | 11.2 |
| 19 | ++--+ --+++ +-+- | 0.12 | 0.10 | 0.05 | 0.005 | 0.10 | 0.05 | 5 | 0.20 | 1.5 | 0.6 | 1.0 | 0.2 | 4 | 5.6 |
| 20 | ++-+- +---- ++-- | 0.12 | 0.10 | 0.05 | 0.010 | 0.05 | 0.30 | 5 | 0.05 | 0.2 | 0.2 | 1.0 | 0.4 | 2 | 5.6 |
| 21 | +++-+ -+--- -++- | 0.12 | 0.10 | 0.10 | 0.005 | 0.10 | 0.05 | 10 | 0.05 | 0.2 | 0.2 | 0.2 | 0.4 | 4 | 5.6 |
| 22 | ++++- +-+-- --++ | 0.12 | 0.10 | 0.10 | 0.010 | 0.05 | 0.30 | 5 | 0.20 | 0.2 | 0.2 | 0.2 | 0.2 | 4 | 11.2 |
| 23 | +++++ +++++ ++++ | 0.12 | 0.10 | 0.10 | 0.010 | 0.10 | 0.30 | 10 | 0.20 | 1.5 | 0.6 | 1.0 | 0.4 | 4 | 11.2 |

**Table 19. Absorbance read-outs indicating biomass concentration after 24 hours of fermentation in the Plackett-Burman experiment**

| Runs | Pattern | Absorbance at 630 nm (a.u.) | | |
|---|---|---|---|---|
| | | Replicate 1 | Replicate 2 | Replicate 3 |
| 1 | ----++--+--+++ | 0.13 | 0.126 | 0.121 |
| 2 | ---++--+--++++ | 0.195 | 0.188 | 0.191 |
| 3 | --+--++++-+-+- | 0.184 | 0.178 | 0.179 |
| 4 | --++--+--++++- | 0.21 | 0.198 | 0.207 |

(continued)

| Runs | Pattern | Absorbance at 630 nm (a.u.) | | |
|------|---------|------------|------------|------------|
| | | Replicate 1 | Replicate 2 | Replicate 3 |
| 5 | --++++-+-+---- | 0.138 | 0.137 | 0.135 |
| 6 | -+----++--+--+ | 0.188 | 0.187 | 0.183 |
| 7 | -+--++++-+-+-- | 0.129 | 0.122 | 0.123 |
| 8 | -+-+----++--+- | 0.108 | 0.112 | 0.113 |
| 9 | -++--+--++++-+ | 0.222 | 0.215 | 0.221 |
| 10 | -++++-+-+----+ | 0.128 | 0.121 | 0.121 |
| 11 | 00000000000000 | 0.175 | 0.163 | 0.165 |
| 12 | 00000000000000 | 0.157 | 0.163 | 0.166 |
| 13 | 00000000000000 | 0.185 | 0.181 | 0.18 |
| 14 | +----++--+--++ | 0.126 | 0.123 | 0.119 |
| 15 | +--+--++++-+-+ | 0.117 | 0.113 | 0.12 |
| 16 | +--++++-+-+--- | 0.195 | 0.203 | 0.2 |
| 17 | +-+----++--+-- | 0.124 | 0.125 | 0.122 |
| 18 | +-+-+----++--+ | 0.223 | 0.213 | 0.212 |
| 19 | ++--+--++++-+- | 0.217 | 0.205 | 0.202 |
| 20 | ++-+-+----++-- | 0.181 | 0.192 | 0.186 |
| 21 | +++-+-+----++- | 0.131 | 0.124 | 0.122 |
| 22 | ++++-+-+----++ | 0.112 | 0.109 | 0.111 |
| 23 | ++++++++++++++ | 0.205 | 0.195 | 0.196 |

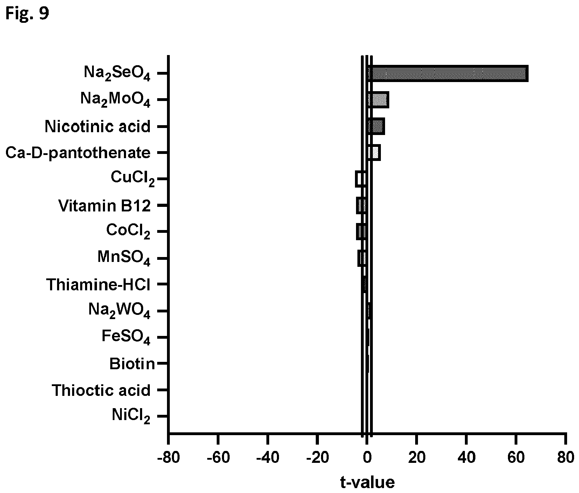

The results of the Plackett-Burman experiment are analysed with JMP (Figure 9). Within the concentrations tested, components containing selenium, molybdenum, nicotinic acid, Ca-D-(+)-pantothenate and tungsten have a significantly positive effect on growth, while components containing copper, vitamin B12, cobalt, manganese and thiamine have a significantly negative effect. Several response surface experiments are performed based off these results, including an analysis on the four components $Na_2SeO_4$, $Na_2MoO_4$, nicotinic acid and Ca-D-(+)-pantothenate.

The response surface experiment is performed as a central composite design with 3 centre points and an axial value of 2 is chosen, and the respective matrix is generated by JMP (Table 20). The high and low values are chosen based on the Plackett-Burman experiment, and the centre and axial points are calculated by JMP. Since all factors have a positive effect in Plackett-Burman, the low levels in RSM are chosen as the high levels from Plackett-Burman, namely 1, 0.6, 0.1 and 0.1 mg/L for $Na_2SeO_4$, $Na_2MoO_4 \cdot 2H_2O$, nicotinic acid and Ca-D-(+)-pantothenate, respectively. The high level is 10 times higher than the low level for each factor, resulting in 10, 6, 1 and 1 mg/L for $Na_2SeO_4$, $Na_2MoO_4 \cdot 2H_2O$, nicotinic acid and Ca-D-(+)-pantothenate, respectively.

**Table 20. Response surface matrix used to optimise the concentrations of $Na_2SeO_4$, $Na_2MoO_4$, nicotinic acid and Ca-D-pantothenate for growth of *Moorella thermoacetica* on $CO_2$**

| Runs | Pattern | $Na_2SeO_4$ (mg/L) | $Na_2MoO_4 \cdot 2H_2O$ (mg/L) | Nicotinic acid (mg/L) | Ca-D-pantothenate (mg/L) |
|------|---------|------------|------------|------------|------------|
| 1 | ---- | 1 | 0.6 | 0.1 | 0.1 |
| 2 | ---+ | 1 | 0.6 | 0.1 | 1.0 |
| 3 | --+- | 1 | 0.6 | 1.0 | 0.1 |
| 4 | --++ | 1 | 0.6 | 1.0 | 1.0 |

(continued)

| Runs | Pattern | Na$_2$SeO$_4$ (mg/L) | Na$_2$MoO$_4$·2H$_2$O (mg/L) | Nicotinic acid (mg/L) | Ca-D-pantothenate (mg/L) |
|---|---|---|---|---|---|
| 5 | -+-- | 1 | 6.0 | 0.1 | 0.1 |
| 6 | -+-+ | 1 | 6.0 | 0.1 | 1.0 |
| 7 | -++- | 1 | 6.0 | 1.0 | 0.1 |
| 8 | -+++ | 1 | 6.0 | 1.0 | 1.0 |
| 9 | +--- | 10 | 0.6 | 0.1 | 0.1 |
| 10 | +--+ | 10 | 0.6 | 0.1 | 1.0 |
| 11 | +-+- | 10 | 0.6 | 1.0 | 0.1 |
| 12 | +-++ | 10 | 0.6 | 1.0 | 1.0 |
| 13 | ++-- | 10 | 6.0 | 0.1 | 0.1 |
| 14 | ++-+ | 10 | 6.0 | 0.1 | 1.0 |
| 15 | +++- | 10 | 6.0 | 1.0 | 0.1 |
| 16 | ++++ | 10 | 6.0 | 1.0 | 1.0 |
| 17 | a000 | 0 | 3.3 | 0.55 | 0.55 |
| 18 | A000 | 14.5 | 3.3 | 0.55 | 0.55 |
| 19 | 0a00 | 5.5 | 0 | 0.55 | 0.55 |
| 20 | 0A00 | 5.5 | 8.7 | 0.55 | 0.55 |
| 21 | 00a0 | 5.5 | 3.3 | 0.00 | 0.55 |
| 22 | 00A0 | 5.5 | 3.3 | 1.45 | 0.55 |
| 23 | 000a | 5.5 | 3.3 | 0.55 | 0.00 |
| 24 | 000A | 5.5 | 3.3 | 0.55 | 1.45 |
| 25 | 0000 | 5.5 | 3.3 | 0.55 | 0.55 |
| 26 | 0000 | 5.5 | 3.3 | 0.55 | 0.55 |
| 27 | 0000 | 5.5 | 3.3 | 0.55 | 0.55 |

The entire procedure of the response surface experiment is performed in an anaerobic chamber. 27 medium solutions are prepared according to the response surface matrix (Table 20), where the concentrations of Na$_2$SeO$_4$, Na$_2$MoO$_4$·2H$_2$O, nicotinic acid and Ca-D-(+)-pantothenate are varied by adding the stock solutions to a medium containing MES monohydrate (final concentration 20 g/L; pH 6.5), NH$_4$Cl (0.4 g/L), KH$_2$PO$_4$ (0.5 g/L), CaCl$_2$·2H$_2$O (0.05 g/L), MgCl$_2$·6H$_2$O (0.33 g/L), NaCl (0.4 g/L), yeast extract (0.5 g/L), biotin (0.12 mg/L), folic acid (0.02 mg/L), pyridoxine-HCl (0.1 mg/L), thiamine-HCl (0.1 mg/L), riboflavin (0.05 mg/L), p-aminobenzoic acid (0.05 mg/L), thioctic acid (0.3 mg/L), vitamin B12 (0.0005 mg/L), KAl(SO$_4$)$_2$·12H$_2$O (0.01 mg/L), CoCl$_2$·6H$_2$O (2 mg/L), CuCl$_2$·2H$_2$O (0.01 mg/L), MnSO$_4$·H$_2$O (7.5 mg/L), FeSO$_4$·7H$_2$O (5.6 mg/L), nitrilotriacetic acid (20 mg/L), Na$_2$WO$_4$·2H$_2$O (0.2 mg/L), ZnSO$_4$·7H$_2$O (2 mg/L), resazurin (0.001 g/L) and cysteine-HCl (1 mM). 490 μL of each solution is aliquoted to 3 wells of a 96-well deep-well plate, and 10 μL inoculum is added to all wells. The plates are placed in an anoxic box flushed with H$_2$/CO$_2$ (80/20) for 10 minutes, with a final pressure of 0.8 bars. The box is incubated at 60 °C without shaking. After 24 hours, the plates are removed from the incubator and each solution is transferred to a 96-well microtiter plate. The absorbance is read in a spectrophotometer at 630 nm (Table 21).

**Table 21. Absorbance read-outs indicating biomass concentration of the RSM experiment on Na$_2$SeO$_4$, Na$_2$MoO$_4$, nicotinic acid and Ca-D-pantothenate**

| Runs | Pattern | Absorbance at 630 nm (a.u.) | | |
|---|---|---|---|---|
| | | Replicate 1 | Replicate 2 | Replicate 3 |
| 1 | ---- | 0.128 | 0.12 | 0.117 |
| 2 | ---+ | 0.116 | 0.114 | 0.113 |
| 3 | --+- | 0.111 | 0.115 | 0.112 |
| 4 | --++ | 0.11 | 0.11 | 0.11 |
| 5 | -+-- | 0.117 | 0.113 | 0.113 |
| 6 | -+-+ | 0.109 | 0.109 | 0.108 |
| 7 | -++- | 0.107 | 0.109 | 0.108 |
| 8 | -+++ | 0.106 | 0.105 | 0.107 |
| 9 | +--- | 0.132 | 0.116 | 0.116 |
| 10 | +--+ | 0.113 | 0.11 | 0.113 |
| 11 | +-+- | 0.111 | 0.11 | 0.109 |
| 12 | +-++ | 0.11 | 0.11 | 0.107 |
| 13 | ++-- | 0.13 | 0.119 | 0.121 |
| 14 | ++-+ | 0.118 | 0.114 | 0.116 |
| 15 | +++- | 0.116 | 0.116 | 0.113 |
| 16 | ++++ | 0.114 | 0.114 | 0.117 |
| 17 | a000 | 0.09 | 0.085 | 0.085 |
| 18 | A000 | 0.118 | 0.116 | 0.114 |
| 19 | 0a00 | 0.118 | 0.118 | 0.118 |
| 20 | 0A00 | 0.114 | 0.113 | 0.112 |
| 21 | 00a0 | 0.128 | 0.12 | 0.118 |
| 22 | 00A0 | 0.117 | 0.112 | 0.115 |
| 23 | 000a | 0.115 | 0.114 | 0.112 |
| 24 | 000A | 0.116 | 0.115 | 0.113 |
| 25 | 0000 | 0.127 | 0.12 | 0.118 |
| 26 | 0000 | 0.115 | 0.114 | 0.117 |
| 27 | 0000 | 0.117 | 0.117 | 0.119 |

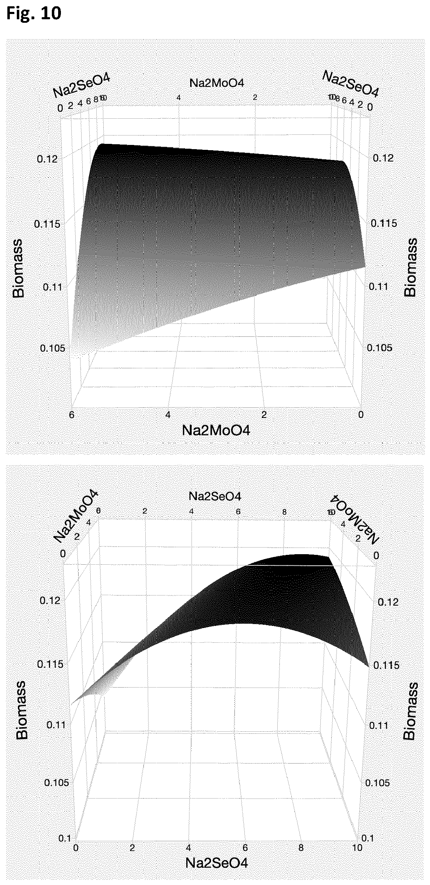

The results are analysed by JMP, which generates a second-degree polynomial equation (Equation 4) and the corresponding response surface plots (Figure 10). The analysis shows that, within the concentrations tested and when $CO_2$ is used as carbon source, biomass concentration increases with decreased Na$_2$MoO$_4$. At the same time, biomass concentration increases with increased Na$_2$SeO$_4$ concentration until a critical level is reached, after which it starts to decline.

$$0.118222222222222 + 0.00352777777777778 * ((Na_2SeO_4 - 5.5) / 4.5) +$$

$$-0.000611111111111111 * ((Na_2MoO_4 - 3.3) / 2.7) + -0.00252777777777778 * ((\text{Nicotinic acid} - 0.55) / 0.45) + -0.00138888888888889 * ((Ca-D-pantothenate}$$

$$-0.55) / 0.45) + (Na_2SeO_4 - 5.5) / 4.5 * (Na_2MoO_4 - 3.3) / 2.7 * 0.00241666666666667$$

$$+ (Na_2SeO_4 - 5.5) / 4.5 * (\text{Nicotinic acid} - 0.55) / 0.45 * -0.000083333333333332$$

$$+(Na_2MoO_4 - 3.3) / 2.7 * (\text{Nicotinic acid} - 0.55) / 0.45 * 0.000583333333333334 + ($$

$$Na_2SeO_4 - 5.5) / 4.5 * (Ca-D-pantothenate - 0.55) / 0.45 * 8.10795832051072e-19$$

$$+ (Na_2MoO_4 - 3.3) / 2.7 * (Ca-D-pantothenate - 0.55) / 0.45 *$$

$$0.000333333333333333 + (\text{Nicotinic acid} - 0.55) / 0.45 * (Ca-D-pantothenate - 0.55$$

$$) / 0.45 * 0.0015 + (Na_2SeO_4 - 5.5) / 4.5 * (Na_2SeO_4 - 5.5) / 4.5 *$$

$$-0.00401388888888889 + (Na_2MoO_4 - 3.3) / 2.7 * (Na_2MoO_4 - 3.3) / 2.7 *$$

$$-0.000472222222222224 + (\text{Nicotinic acid} - 0.55) / 0.45 * (\text{Nicotinic acid} - 0.55) /$$

$$0.45 * 0.00023611111111111 + (Ca-D-pantothenate - 0.55) / 0.45 * ($$

$$Ca-D-pantothenate - 0.55) / 0.45 * -0.000805555555555556$$

**Equation 4. Second degree polynomial equation resulted from the RSM experiment on $Na_2SeO_4$, $Na_2MoO_4$, nicotinic acid and Ca-D-pantothenate**

**Example 6**

This example describes the improved biomass production of *Moorella thermoacetica* in a growth medium containing novel concentrations of certain nutrients when $CO_2$ is used as carbon source.

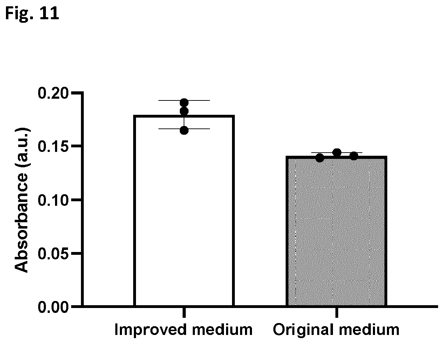

The procedure is performed in an anaerobic chamber to ensure an anoxic environment. As per Examples 4 and 5, sterile and anaerobic stock solutions of each component are prepared. Two media, termed "original" and "improved" media, are prepared by mixing stock solutions to reach the concentrations shown in Table 22. 490 $\mu$L of each solution is aliquoted to 3 wells of a 96-well deep-well plate, and 10 $\mu$L inoculum is added to all wells. The plates are placed in an anoxic box flushed with $H_2/CO_2$ (80/20) for 10 minutes, with a final pressure of 0.8 bars. The box is incubated at 60 °C without shaking. After 24 hours, the plates are removed from the incubator and each solution is transferred to a 96-well microtiter plate. The absorbance is read in a spectrophotometer at 630 nm (Figure 11). Accordingly, the improved medium shows an average 27 % increase in biomass concentration compared to the original medium.

**Table 22. Concentrations of medium components in the "original" and "improved" media using $CO_2$ as carbon source for fermentation with *Moorella thermoacetica***

| Component | Concentration in "original medium" | Concentration in "improved medium" |
|---|---|---|
| MES monohydrate (g/L) | 20 | 20 |

(continued)

| Component | Concentration in "original medium" | Concentration in "improved medium" |
|---|---|---|
| Resazurin (g/L) | 0.001 | 0.001 |
| Cysteine-HCl (mM) | 1 | 1 |
| Yeast extract (g/L) | 0.5 | 0.5 |
| $NH_4Cl$ (g/L) | 0.4 | 0.2 |
| $MgCl_2 \cdot 6H_2O$ (g/L) | 0.33 | 4.5 |
| $CaCl_2 \cdot 2H_2O$ (g/L) | 0.05 | 0.05 |
| $KH_2PO_4$ (g/L) | 0.5 | 0.01 |
| NaCl (g/L) | 0.4 | 1 |
| Biotin (mg/L) | 0.02 | 0.12 |
| Folic acid (mg/L) | 0.02 | 0.02 |
| Pyridoxine-HCl (mg/L) | 0.1 | 0.1 |
| Thiamine-HCl (mg/L) | 0.05 | 0.1 |
| Riboflavin (mg/L) | 0.05 | 0.05 |
| Nicotinic acid (mg/L) | 0.05 | 0.1 |
| Ca-D-Pantothenate (mg/L) | 0.05 | 0.1 |
| Vit B12 (mg/L) | 0.005 | 0.004 |
| p-aminobenzoic acid (mg/L) | 0.05 | 0.05 |
| Thioctic acid (mg/L) | 0.05 | 0.3 |
| $CoCl_2 \cdot 6H_2O$ (mg/L) | 2 | 0.2 |
| $CuCl_2 \cdot 2H_2O$ (mg/L) | 0.2 | 0.01 |
| $FeSO_4 \cdot 7H_2O$ (mg/L) | 5.6 | 5.6 |
| $MnSO_4 \cdot H_2O$ (mg/L) | 10 | 3 |
| $Na_2MoO_4 \cdot 2H_2O$ (mg/L) | 0.2 | 0.6 |
| $NiCl_2 \cdot 6H_2O$ (mg/L) | 0.2 | 1.5 |
| Nitrilotriacetic acid (mg/L) | 20 | 20 |
| $Na_2SeO_4$ (mg/L) | 0.2 | 7.5 |
| $Na_2WO_4 \cdot 2H_2O$ (mg/L) | 0.2 | 0.2 |
| $ZnSO_4 \cdot 7H_2O$ (mg/L) | 2 | 2 |

## Claims

1. A method for increasing the growth efficiency in an anaerobic microbial fermentation of a microorganism capable of converting $CO_2$, CO or a mixture thereof into at least one organic compound, the method comprising growing the microorganisms in a cultivation medium comprising selenium in a concentration which is at least 1.2 $\mu$M.

2. A method for increasing the growth efficiency in an anaerobic microbial fermentation of a microorganism converting $CO_2$, CO or a mixture thereof into at least one organic compound, the method comprising growing the microorganisms in a cultivation medium comprising nickel in a concentration which is in the range from 3.0 $\mu$M to 8.5 $\mu$M.

3. A method for increasing the growth efficiency in an anaerobic microbial fermentation of a microorganism converting $CO_2$, CO or a mixture thereof into at least one organic compound, the method comprising growing the microorganisms in a cultivation medium comprising molybdenum in a concentration which is at least 1.5 $\mu$M.

4. The method according to any of the preceding claims, wherein said cultivation medium comprises selenium in a concentration which is at least 1.3 $\mu$M, at least 2 $\mu$M, at least 3 $\mu$M, or at least 4 $\mu$M.

5. The method according to any of the preceding claims, wherein said cultivation medium comprises selenium in a concentration which is in the range from 2.0 $\mu$M to 8 $\mu$M, in the range from 3.0 $\mu$M to 7 $\mu$M, in the range from 4 $\mu$M to 6 $\mu$M or in the range from 4.4 $\mu$M to 5.6 $\mu$M.

6. The method according to any of the preceding claims, wherein said cultivation medium comprises nickel in a concentration which is in the range from 4.0 $\mu$M to 8.0 $\mu$M, in the range from 5.0 $\mu$M to 7.0 $\mu$M or in the range from 5.5 $\mu$M to 7.0 $\mu$M.

7. The method according to any of the preceding claims, wherein said cultivation medium comprises molybdenum in a concentration which is at least 1.7 $\mu$M, at least 2.0 $\mu$M, or at least 2.2 $\mu$M.

8. The method according to any of the preceding claims, wherein said microorganism is capable of converting $CO_2$ into at least one organic compound.

9. The method according to any of the preceding claims, wherein said microorganism is capable of converting CO into at least one organic compound.

10. The method according to any of the preceding claims, wherein said microorganism is an acetogen.

11. The method according to any of the preceding claims, wherein said microorganism is selected from the group consisting of *Clostridium, Moorella, Thermoonoerobocter, Thermoanaerobacterium, Acetogenium, Acetobocterium, Acetoonoerobium, Butyribocterium, Eubacterium, Pyrococcus, Desulfobacterium* and *Carboxydothermus.*

12. The method according to any of the preceding claims, wherein said at least one organic compound comprises acetate.

13. The method according to any of the preceding claims, wherein said anaerobic microbial fermentation is a production of said at least one organic compound from said cultivation medium and a gas comprising $CO_2$, CO or a mixture thereof.

14. Use of the method as defined in any of claims 1-13 for the industrial manufacture of at least one organic compound from $CO_2$, CO or a mixture thereof.

15. The use according to claim 14, wherein said at least one organic compound comprises acetate.

**Fig. 1**

# Fig. 2

Fig. 3

## Fig. 4

**Fig. 5**

Cell density (a.u.) vs Time (h)

--- Improved medium
— Original medium

**Fig. 6**

Acetate (g/L)

Improved medium    Original medium

**Fig. 7**

**Fig. 8**

**Fig. 9**

**Fig. 10**

Fig. 11

Europäisches
Patentamt

European
Patent Office

Office européen
des brevets

# EUROPEAN SEARCH REPORT

**Application Number**

EP 22 19 9963

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/300593 A1 (MIHALCEA CHRISTOPHE DANIEL [NZ] ET AL) 8 December 2011 (2011-12-08) | 1-15 | INV. C12P7/06 C12P7/54 C05D9/02 C12N1/20 C12R1/145 |
| Y | * pages 1-3,7 * | 2,5,6 | |
| X | US 2020/071734 A1 (SENARATNE RYAN H [US] ET AL) 5 March 2020 (2020-03-05) | 1-15 | |
| Y | * page 1 - page 5 * | 2,5,6 | |
| X | JYOTISNA SAXENA ET AL: "Effect of trace metals on ethanol production from synthesis gas by the ethanologenic acetogen, Clostridium ragsdalei", JOURNAL OF INDUSTRIAL MICROBIOLOGY & BIOTECHNOLOGY, vol. 38, no. 4, 1 April 2011 (2011-04-01), pages 513-521, XP055086240, ISSN: 1367-5435, DOI: 10.1007/s10295-010-0794-6 | 1-15 | |
| Y | * the whole document * | 2,5,6 | |
| X | SUN XIAO ET AL: "Syngas fermentation process development for production of biofuels and chemicals: A review", BIORESOURCE TECHNOLOGY REPORTS, vol. 7, 6 July 2019 (2019-07-06), page 100279, XP055782629, GB ISSN: 2589-014X, DOI: 10.1016/j.biteb.2019.100279 | 1-15 | |
| Y | * page 1 - page 3; table 2 * | 2,5,6 | |

-/--

**TECHNICAL FIELDS SEARCHED (IPC)**

C12P
C05G
C05D
C12R
C12N

The present search report has been drawn up for all claims

| Place of search | Date of completion of the search | Examiner |
|---|---|---|
| Berlin | 8 March 2023 | Schneider, Patrick |

EPO FORM 1503 03.82 (P04C01)

page 1 of 2

Europäisches
Patentamt

European
Patent Office

Office européen
des brevets

## EUROPEAN SEARCH REPORT

Application Number

EP 22 19 9963

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHEN SHAOHUANG ET AL:  "Enhanced alcohol titre and ratio in carbon monoxide-rich off-gas fermentation ofClostridium carboxidivoransthrough combination of trace metals optimization with variable-temperature cultivation", BIORESOURCE TECHNOLOGY, ELSEVIER, AMSTERDAM, NL, vol. 239, 27 April 2017 (2017-04-27), pages 236-243, XP085058838, ISSN: 0960-8524, DOI: 10.1016/J.BIORTECH.2017.04.099 | 1,2,4-6, 8-15 | |
| Y | * page 236 – pages 238,241 * | 2,5,6 | |
| A | ----- | 3,7 | |

TECHNICAL FIELDS SEARCHED (IPC)

The present search report has been drawn up for all claims

| Place of search | Date of completion of the search | Examiner |
|---|---|---|
| Berlin | 8 March 2023 | Schneider, Patrick |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

EP 4 349 993 A1

## ANNEX TO THE EUROPEAN SEARCH REPORT
## ON EUROPEAN PATENT APPLICATION NO.

EP 22 19 9963

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2023

| Patent document cited in search report | Publication date | Patent family member(s) | Publication date |
|---|---|---|---|
| US 2011300593 A1 | 08-12-2011 | US 2011300593 A1 | 08-12-2011 |
| | | US 2013230894 A1 | 05-09-2013 |
| | | WO 2010064933 A1 | 10-06-2010 |
| US 2020071734 A1 | 05-03-2020 | AU 2019319671 A1 | 14-01-2021 |
| | | AU 2019319672 A1 | 14-01-2021 |
| | | BR 112021000261 A2 | 06-04-2021 |
| | | BR 112021000355 A2 | 06-04-2021 |
| | | CA 3106325 A1 | 13-02-2020 |
| | | CA 3106326 A1 | 13-02-2020 |
| | | CN 112689680 A | 20-04-2021 |
| | | CN 112771170 A | 07-05-2021 |
| | | EP 3833771 A1 | 16-06-2021 |
| | | EP 3833772 A1 | 16-06-2021 |
| | | JP 2021532805 A | 02-12-2021 |
| | | JP 2021532806 A | 02-12-2021 |
| | | KR 20210042082 A | 16-04-2021 |
| | | KR 20210042083 A | 16-04-2021 |
| | | TW 202012630 A | 01-04-2020 |
| | | TW 202012632 A | 01-04-2020 |
| | | US 2020071734 A1 | 05-03-2020 |
| | | US 2020308611 A1 | 01-10-2020 |
| | | WO 2020033261 A1 | 13-02-2020 |
| | | WO 2020033262 A1 | 13-02-2020 |

EPO FORM P0459

For more details about this annex : see Official Journal of the European Patent Office, No. 12/82

**EP 4 349 993 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9837179 A
- WO 2010064932 A
- US 20150024449 A

**Non-patent literature cited in the description**

- **REDL et al.** *Frontiers in Microbiology,* 2020, vol. 10, 1-15